(19) Europäisches Patentamt
European Patent Office
Office européen des brevets

(11) Publication number: 0 385 775 A1

# (12) EUROPEAN PATENT APPLICATION

(21) Application number: **90302188.9**

(22) Date of filing: **01.03.90**

(51) Int. Cl.5: **C07D 413/12, A01N 47/36, C07D 417/12, C07D 403/12, C07D 265/36**

(30) Priority: **03.03.89 AU 3050/89**

(43) Date of publication of application: **05.09.90 Bulletin 90/36**

(84) Designated Contracting States: **AT BE CH DE DK ES FR GB GR IT LI LU NL SE**

(71) Applicant: **ICI AUSTRALIA LIMITED**
**1 Nicholson Street**
**Melbourne Victoria 3001(AU)**

(72) Inventor: **Anderson, Janet**
**1 McAuley Drive**
**Rosanna 3084, Victoria(AU)**
Inventor: **Bell, Stephen**
**18 Mirams Street**
**Ascot Vale 3032, Victoria(AU)**
Inventor: **Best, Wayne**
**4/4 Carmyle Court**
**Avondale Heights 3034, Victoria(AU)**
Inventor: **Watson, Keith**
**18 Hamilton Avenue**
**Blackburn 3130, Victoria(AU)**
Inventor: **Drygala, Peter**
**F205/1 Dick Ward Drive**
**Fannie Bay 0820, Northern Territory(AU)**

(74) Representative: **Hardman, Carol Pauline et al**
**Imperial Chemical Industries PLC Legal Department: Patents PO Box 6 Bessemer Road**
**Welwyn Garden City Herts, AL7 1HD(GB)**

## (54) Herbicidal sulfonamides.

(57) Compounds of the formula

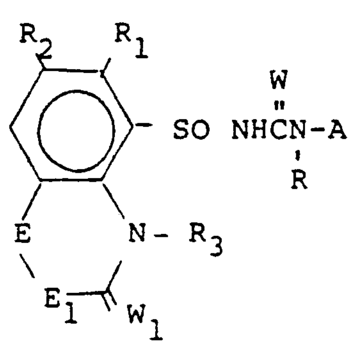

and salts thereof, W and $W_1$ being independently O or S; A being a nitrogen-containing heterocyclic ring system, E being O, $S(O)_m$ or $NR_4$ where m is 0-2; $E_1$ being $CH_2$, $CH_2CH_2$, CH ($C_1$-$C_4$ alkyl), $C(C_1$-$C_4$ alkyl$)_2$ or CH (aryl); $R_1$ and $R_2$ are independently hydrogen, halogen or one of a variety of organic substituents. The compounds are effective herbicides.

EP 0 385 775 A1

## HERBICIDAL SULFONAMIDES

This invention relates to organic compounds having biological activity and in particular to organic compounds having herbicidal properties and plant growth regulating properties, to processes for the preparation of such compounds, to intermediates useful in the preparation of such compounds and to herbicidal compositions and processes utilizing such compounds.

The use of certain sulfonylurea derivatives as herbicides is known in the art. Thus, for example, the "Pesticide Manual" (C R Worthing Editor, The British Crop Protection Council, 7th Edition 1983) describes the sulfonylurea derivative known commercially as chlorsulfuron [1-(2-chlorophenylsulfonyl)-3-(4-methoxy-6-methyl-1,3,5-triazin-2-yl) urea] and its use as a broadleaf weed herbicide in cereals. This compound is described in Australian Patent No. 510,056 and its equivalents such as U.S. Patent 4,127,405.

Australian Patent Application No. 89,354/82 (published April 21, 1983) discloses herbicidal benzenesulfonylureas including those of general formulae I and II :

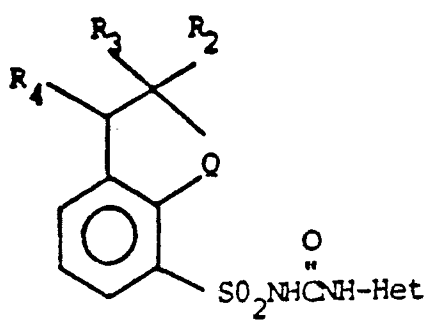

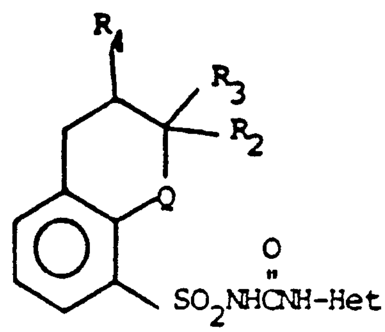

wherein
X as Q is O, S or $SO_2$;
$R_2$ is H or $C_1$ to $C_3$ alkyl;
$R_3$ and $R_4$ are H or $CH_3$;
Het is a pyrimidyl or triazinyl heterocyclic ring.

European Patent Application 82,681 (published June 29, 1983) discloses herbicidal benzenesulfonylureas including those of general formulae III and IV:

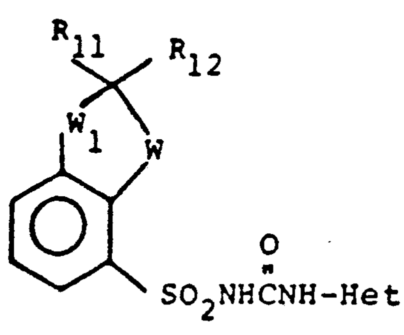

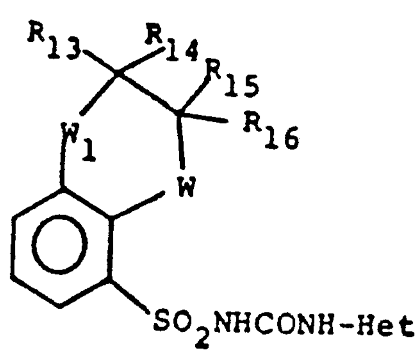

Wherein:
W and $W_1$ are O, S, SO or $SO_2$;
$R_{11}$ is H or $CH_3$;
$R_{12}$ is H or $C_1$ to $C_4$ alkyl;
$R_{13}$, $R_{14}$, $R_{15}$ and $R_{16}$ are independently H or $CH_3$;
Het is a pyrimidyl or triazinyl heterocyclic ring.

Australian Patent Application No. 20659/83 (published May 3, 1984) discloses herbicidal sulfonylureas including those of general formulae V and VI :

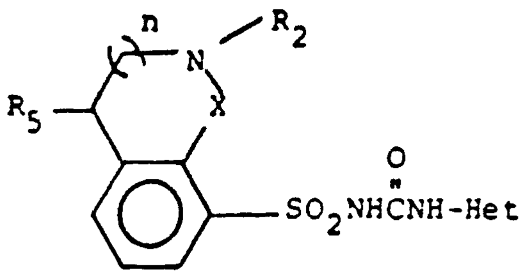

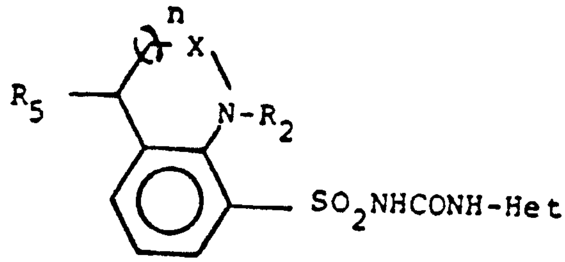

wherein
X is C=O or $SO_2$;
$R_2$ is H or $C_1$ to $C_4$ alkyl;
$R_5$ is H or $CH_3$;
n is 0, 1 or 2.

Australian Patent Application No. 16889/83 (published January 19, 1984) discloses herbicidal sulfonylureas of the general structure VII :

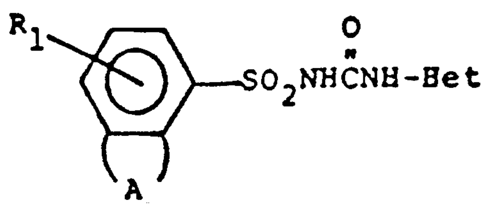

wherein
A is an unsubstituted or substituted bridge of 3 or 4 atoms which contains 1 or 2 oxygen, sulfur or nitrogen atoms and, together with the linking carbon atom, forms a non-aromatic 5- or 6-membered heterocyclic ring system, with the proviso that two oxygen atoms are separated by at least one carbon atom and that oxygen and sulfur atoms are only linked to each other if the sulfur atom takes the form of the -SO- or $SO_2$- group;
$R_1$ is hydrogen, halogen, $NO_2$, $C_1$-$C_4$ alkyl, $C_1$-$C_4$ haloalkyl, $C_1$-$C_4$ alkoxy, $C_1$-$C_4$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy; Het is a pyrimidyl or triazinyl heterocyclic ring.

Australian Patent Application No. 54,625/86 (published October 16, 1986) discloses herbicidal sulfonylureas including those of the general structure VIII :

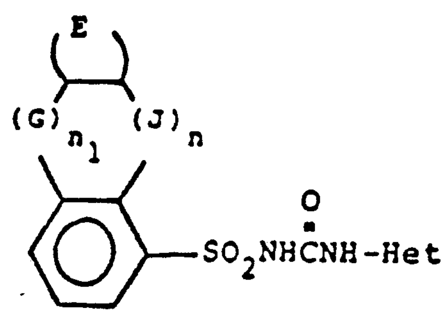

wherein
G is $CH_2$, $CH_2CH_2$, O, S, NH, $NCH_3$ or CH=CH;
J is $CH_2$, C=O, $S(O)_m$, O, NH, $NCH_3$, CHOH, $CHOCH_3$, $CH(CH_3)$ or $C(CH_3)OH$; n and $n_1$ are independently 0 or 1, m is 0, 1 or 2; E is a bridge of 3 or 4 atoms containing 0 to 2 heteroatoms; Het is a pyrimidyl or triazinyl heterocyclic ring.

Australian Patent Application No. 23,771/88 (filing date October 13, 1988) discloses herbicidal sulfonylureas including those of the general structure X:

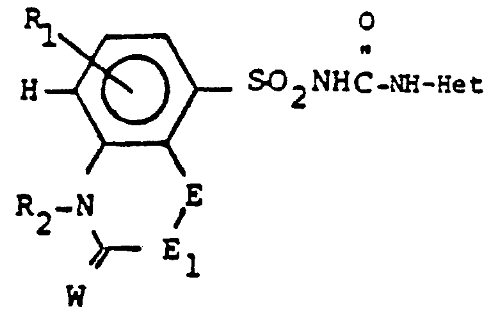

wherein
W is O or S;
E is O, $S(O)_m$ or N-$R_3$;
$E_1$ is $CH_2$, $CH_2CH_2$, CH($C_1$-$C_4$ alkyl) or $C(CH_3)_2$;
$R_1$ is hydrogen, halogen, $C_1$-$C_4$ alkyl, $C_1$-$C_4$ haloalkyl, $C_1$-$C_4$ alkoxy, $C_1$-$C_4$ halo-alkoxy, $C_1$-$C_4$ alkoxycarbonyl, $C_1$-$C_4$ alkylthio, $C_1$-$C_4$ alkylsulfinyl, $C_1$-$C_4$ alkylsulfonyl, sulfamoyl, $C_1$-$C_4$ alkylsulfamoyl, di($C_1$-$C_4$ alkyl)sulfamoyl, amino, $C_1$-$C_4$ alkylamino, or di($C_1$-$C_4$ alkyl) amino;
R, $R_2$ and $R_3$ are independently hydrogen, $C_1$-$C_4$ alkyl, $C_1$-$C_4$ alkenyl or $C_1$-$C_4$ alkynyl;
m = 0, 1 or 2;
Het is a pyrimidyl or triazinyl heterocyclic ring.

## Summary of the Invention

It has now been found that a new group of bicyclic sulfonylurea derivatives exhibit particularly useful herbicidal activity. They have both pre-emergent and post-emergent application, and some show selective herbicidal activity against broad-leaved and grass weeds in important crops such as maize, rice, wheat, barley, sugarbeet, cotton and soya.

Accordingly the invention provides compounds of the formula I

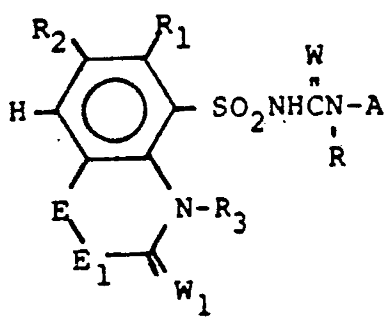

I

wherein
W and $W_1$ are independently O or S;
E is O, $S(O)_m$, or N-$R_4$;
E is $CH_2$, $CH_2CH_2$, CH($C_1$-$C_4$ alkyl), C($C_1$-$C_4$ alkyl)$_2$ or CH (aryl);
$R_1$ and $R_2$ are independently hydrogen, halogen, $C_1$-$C_4$ alkyl, $C_1$-$C_4$ haloalkyl, arylalkyl, nitro, $C_1$-$C_4$ alkoxy, $C_1$-$C_4$ haloalkoxy, cyano, carboxy, carbamoyl, N-($C_1$-$C_4$ alkyl)carbamoyl, N,N-di($C_1$-$C_4$ alkyl)carbamoyl, $C_1$-$C_4$ alkoxycarbonyl, $C_1$-$C_4$ alkylthio, $C_1$-$C_4$ alkylsulfinyl, $C_1$-$C_4$ alkylsulfonyl, sulfamoyl, $C_1$-$C_4$ alkylsulfamoyl, di($C_1$-$C_4$ alkyl) sulfamoyl, amino, $C_1$-$C_4$ alkylamino or di($C_1$-$C_4$ alkyl) amino, $C_1$-$C_4$ acylamino;
R, $R_3$ and $R_4$ are each independently selected from hydrogen, optionally substituted $C_1$-$C_4$ alkyl, $C_2$-$C_4$ alkenyl, $C_2$-$C_4$ alkynyl, or optionally substituted aryl or arylalkyl; and m = 0, 1 or 2;

A is 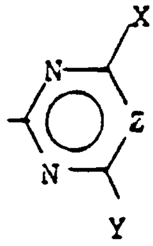, 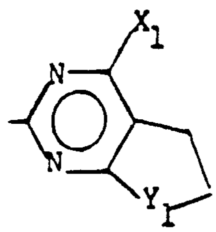, 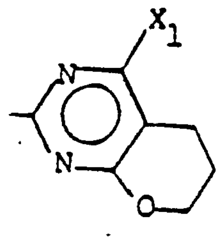, 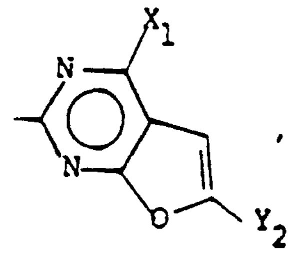,

A-1 A-2 A-3 A-4

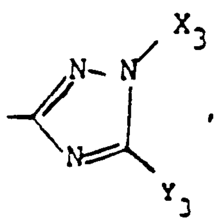, 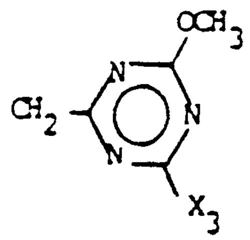 or 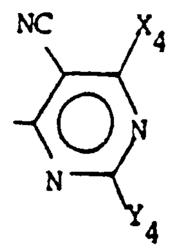

A-5 A-6 A-7

X is $CH_3$, $OCH_3$, $OCH_2CH_3$, Cl, F, Br, I, $OCF_2H$, $CH_2F$ or $CF_3$;
Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $N(OCH_3)CH_3$, $OCH_2CH=CH_2$, $OCH_2C\equiv CH$, $OCH_2CF_3$, cyclopropyl, $OCH_2CH_2OCH_3$, $CH_2SCH_3$, $(C=O)R_4$, $CR_4(QCH_3)_2$,

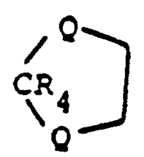,

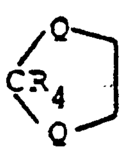, 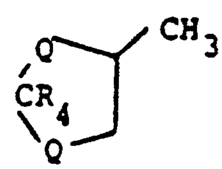

$CR_4(QCH_2CH_3)_2$, $OCF_2H$, $SCF_2H$, $C\equiv CH$ or $C\equiv CCH_3$ where Q is O or S and $R_4$ is H or $CH_3$;
Z is CH, H, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$Y_2$ is H or $CH_3$;
$X_2$ is $CH_3$, $C_2H_5$ or $CH_2CF_3$;
$Y_3$ is $OCH_3$, $OC_2H_5$, $SCH_3$, $SC_2H_5$, $CH_3$ or $CH_2CH_3$;
$X_3$ is $CH_3$ or $OCH_3$;
$Y_4$ is $CH_3$, $OCH_3$, $OCH_2CH_3$ or Cl; and
$X_4$ is $CH_3$, $OCH_3$, $OCH_2CH_3$, $CH_2OCH_3$ or Cl.

The invention also comprises the salts which the compounds of formula 1 are able to form with amines, alkali metal bases and alkaline earth metal bases, or with quaternary ammonium bases.

In the above definitions, the term "alkyl" used either alone or in compound words such as "alkylthio" or

"haloalkyl", denotes straight chain or branched alkyl, e.g., methyl, ethyl, n-propyl, isopropyl or the different butyl isomers.

The term "aryl" used either alone or in the word "arylalkyl" denotes an aromatic ring such as phenyl or pyridyl, optionally substituted by halogen, methoxy, methyl, trifluoromethyl or nitro groups.

Alkoxy denotes methoxy, ethoxy, n-propoxy, isopropoxy and the different butyl isomers.

Alkenyl denotes straight chain or branched alkenes, e.g., vinyl, 1-propenyl, 2-propenyl, 3-propenyl or the different butenyl isomers.

Alkylsulfonyl denotes methylsulfonyl, ethylsulfonyl or the different propylsulfonyl isomers.

Alkylthio, alkylsulfinyl, alkylamino, etc. are defined in an analogous manner.

Preferred compounds of the invention include:

1) Those compounds of formula I where

W is O and A is A-1.

2) Compounds of Preferred 1) where

R is hydrogen;

$R_1$ and $R_2$ are independently hydrogen, fluorine, chlorine, bromine, methyl, methoxy, trifluoromethyl, methoxycarbonyl or methylthio;

and Y is methyl, methoxy, ethoxy, methoxymethyl, trifluoromethyl, 2,2,2-trifluoroethoxy, dimethoxymethyl or difluoromethoxy.

A further group of preferred compounds of formula I comprises those compounds wherein :

E is O, S, NH or $NCH_3$;

$E_1$ is $CH_2$, $CH_2CH_2$, or $CH(CH_3)$;

$W_1$ is O, and $R_3$ is methyl or ethyl.

Examples of the basic types of condensed saturated heterocyclic systems which are attached to the sulfonyl end of the sulfonylurea bridge are:

3-oxo-3,4-dihydro-2H-1,4-benzoxazines $B_1$,

3-oxo-3,4-dihydro-2H-1,4-benzothiazines $B_2$,

2-oxo-1,2,3,4-tetrahydroquinoxalines $B_3$,

2,3-dihydro-1,5-benzoxazepin-4-ones $B_4$,

2,3-dihydro-1,5-benzothiazepin-4-ones $B_5$, and

3,4-dihydro-1,5-benzodiazepine-2-ones $B_6$.

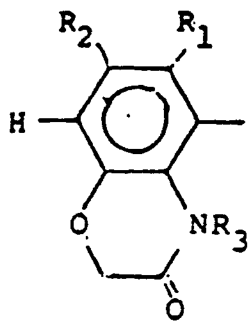

$B_1$

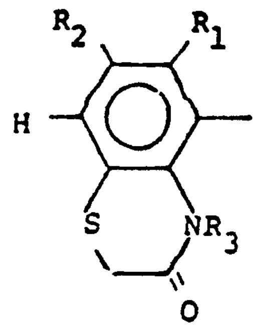

$B_2$

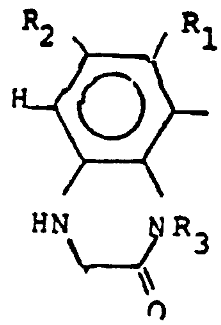

$B_3$

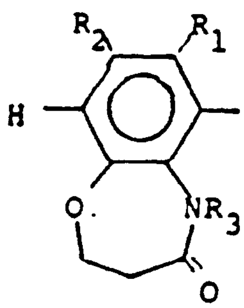

$B_4$

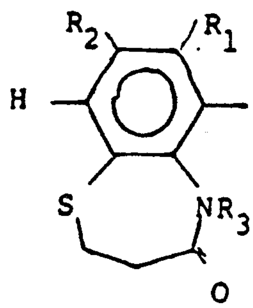

$B_5$

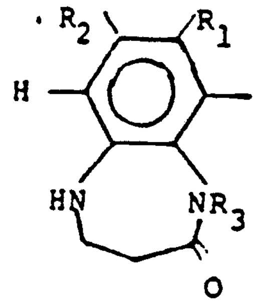

$B_6$

Specific examples of the compounds of the invention include those compounds listed in Tables 1a - 1d.

TABLE Ia

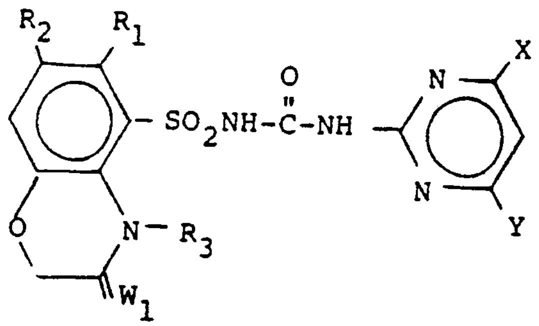

| Compound No. | $R_1$ | $R_2$ | $R_3$ | $W_1$ | X | Y |
|---|---|---|---|---|---|---|
| 1 | H | H | H | O | Me | OMe |
| 2 | H | H | H | O | OMe | OMe |
| 3 | H | H | H | S | Me | OMe |
| 4 | H | H | H | S | OMe | OMe |
| 5 | H | H | Me | O | Me | Me |
| 6 | H | H | Me | O | Me | OMe |
| 7 | H | H | Me | O | OMe | OMe |
| 8 | H | H | Me | O | Cl | OMe |
| 9 | H | H | Me | O | Me | OEt |
| 10 | H | H | Me | O | Cl | NHMe |
| 11 | H | H | Me | O | $CF_2H$ | $OCF_2H$ |
| 12 | H | H | Me | O | Cl | $NMe_2$ |
| 13 | H | H | Me | O | Cl | $NH_2$ |
| 14 | H | H | Me | O | Me | SMe |
| 15 | H | H | Me | O | Me | $CH_2OMe$ |
| 16 | H | H | Me | O | $CF_3$ | OMe |
| 17 | H | H | Me | O | OMe | $OCF_2H$ |
| 18 | H | H | Me | O | OMe | $NMe_2$ |
| 19 | H | H | Me | S | Me | OMe |
| 20 | H | H | Me | S | OMe | OMe |
| 21 | H | H | Me | S | $OCF_2H$ | $OCF_2H$ |
| 22 | H | H | Me | S | Cl | OMe |

Table 1a (Continued)

| Compound No. | $R_1$ | $R_2$ | $R_3$ | $W_1$ | X | Y |
|---|---|---|---|---|---|---|
| 23 | H | H | Et | O | Me | OMe |
| 24 | H | H | Et | O | OMe | OMe |
| 25 | H | H | Et | O | Cl | OMe |
| 26 | H | H | Et | O | $OCF_2H$ | $OCF_2H$ |
| 27 | H | H | Et | Me | OMe | OMe |
| 28 | H | H | Et | S | OMe | OMe |
| 29 | H | H | Allyl | O | Me | OMe |
| 30 | H | H | Allyl | O | OMe | OMe |
| 31 | H | H | nPr | O | Me | OMe |
| 32 | H | H | nPr | O | OMe | OMe |
| 33 | H | H | Benzyl | O | Me | OMe |
| 34 | H | H | Benzyl | O | OMe | OMe |
| 35 | Br | H | H | O | Me | OMe |
| 36 | Br | H | H | O | OMe | OMe |
| 37 | Br | H | Me | O | Me | OMe |
| 38 | Br | H | Me | O | OMe | OMe |
| 39 | Br | H | Me | O | Cl | OMe |
| 40 | Br | H | Me | O | $OCF_2H$ | $OCF_2H$ |
| 41 | Br | H | Me | S | Me | OMe |
| 42 | Br | H | Et | O | Me | OMe |
| 43 | Cl | H | Me | O | Me | OMe |
| 44 | Cl | H | Me | O | OMe | OMe |
| 45 | Cl | H | Me | S | Me | OMe |
| 46 | Cl | H | Et | O | Me | OMe |
| 47 | Me | H | Me | O | Me | OMe |
| 48 | Me | H | Me | S | Me | OMe |
| 49 | Me | H | Et | O | Me | OMe |
| 50 | Me | Me | Me | O | Me | OMe |
| 51 | Me | Me | Me | S | Me | Me |
| 52 | Me | Me | Et | O | Me | OMe |

Table 1a (Continued)

| Compound No. | $R_1$ | $R_2$ | $R_3$ | $W_1$ | X | Y |
|---|---|---|---|---|---|---|
| 53 | $CO_2Me$ | H | Me | O | Me | OMe |
| 54 | $CO_2Me$ | H | Me | O | OMe | OMe |
| 55 | $CO_2Me$ | H | Me | O | Cl | OMe |
| 56 | $CO_2Me$ | H | Me | S | Me | OMe |
| 57 | $CO_2Me$ | H | Et | O | Me | OMe |
| 58 | $CO_2Me$ | $C_1$ | Me | O | Me | OMe |
| 59 | $CONMe_2$ | H | Me | O | Me | OMe |
| 60 | $CONMe_2$ | H | Me | O | OMe | OMe |
| 61 | $CONMe_2$ | H | Me | O | Cl | OMe |
| 62 | $CONMe_2$ | H | Me | S | Me | OMe |
| 63 | $CONMe_2$ | H | Et | O | Me | OMe |
| 64 | OMe | H | Me | O | Me | OMe |
| 65 | OMe | H | Me | O | OMe | OMe |
| 66 | OMe | H | Me | S | Me | OMe |
| 67 | OMe | H | Et | O | Me | OMe |
| 68 | SMe | H | Me | O | Me | OMe |
| 69 | $CF_3$ | H | Me | O | Me | OMe |

TABLE Ib

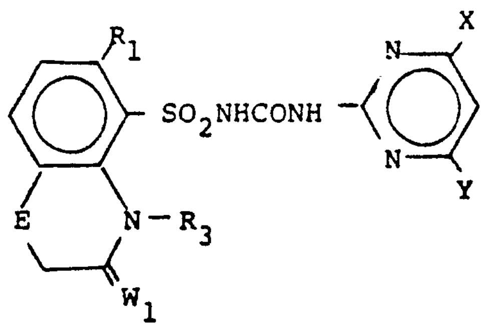

| Compound No. | $R_1$ | $R_2$ | $R_3$ | $W_1$ | X | Y |
|---|---|---|---|---|---|---|
| 70 | H | H | S | O | Me | OMe |
| 71 | H | Me | S | O | Me | OMe |
| 72 | H | Me | S | O | OMe | OMe |
| 73 | H | Me | S | O | Cl | OMe |
| 74 | H | Me | S | O | $OCF_2H$ | $OCF_2H$ |
| 75 | H | Me | S | S | Me | OMe |
| 76 | H | Et | S | O | Me | OMe |
| 77 | Cl | Me | S | O | Me | OMe |
| 78 | Me | Me | S | O | Me | OMe |
| 79 | OMe | Me | S | O | Me | OMe |
| 80 | $CO_2Me$ | Me | S | O | Me | OMe |
| 81 | $CONMe_2$ | Me | S | O | Me | OMe |
| 82 | H | H | SO | O | Me | OMe |
| 83 | H | Me | SO | O | Me | OMe |
| 84 | H | Me | SO | O | Cl | OMe |
| 85 | H | Me | SO | O | $OCF_2H$ | $OCF_2H$ |
| 86 | H | Me | SO | S | Me | OMe |
| 87 | H | Me | SO | O | Me | OMe |
| 88 | H | Et | SO | O | Me | OMe |
| 89 | Cl | H | SO | O | Me | OMe |
| 90 | Me | Me | SO | O | Me | OMe |
| 91 | OMe | Me | SO | O | Me | OMe |

TABLE 1b (Continued)

| Compound No. | $R_1$ | $R_2$ | $R_3$ | $W_1$ | X | Y |
|---|---|---|---|---|---|---|
| 92 | $CO_2Me$ | Me | SO | O | Me | OMe |
| 93 | $CONMe_2$ | Me | SO | O | Me | OMe |
| 94 | H | H | $SO_2$ | O | OMe | OMe |
| 95 | H | Me | $SO_2$ | O | Cl | OMe |
| 96 | H | Me | $SO_2$ | O | $OCF_2H$ | $OCF_2H$ |
| 97 | H | Me | $SO_2$ | O | Me | OMe |
| 98 | H | Me | $SO_2$ | O | OMe | OMe |
| 99 | H | Me | $SO_2$ | S | Me | OMe |
| 100 | H | Et | $SO_2$ | O | Me | OMe |
| 101 | Cl | Me | $SO_2$ | O | Me | OMe |
| 102 | Me | Me | $SO_2$ | O | Me | OMe |
| 103 | $CO_2Me$ | Me | $SO_2$ | O | Me | OMe |
| 104 | $CONME_2$ | Me | $SO_2$ | O | Me | OMe |
| 105 | H | H | NH | O | Me | OMe |
| 106 | H | Me | NH | O | Me | OMe |
| 107 | H | Me | NH | S | Me | OMe |
| 108 | H | Et | NH | O | Me | OMe |
| 109 | H | H | NMe | O | Me | OMe |
| 110 | H | Me | NMe | O | Me | OMe |
| 111 | H | Me | NMe | O | Me | OMe |
| 112 | H | Me | NMe | S | Me | OMe |
| 113 | H | Et | NMe | O | Me | OMe |
| 114 | Cl | Me | NMe | O | Me | OMe |
| 115 | Me | Me | NMe | O | Me | OMe |
| 116 | $CO_2Me$ | Me | NMe | O | Me | OMe |
| 117 | $CONMe_2$ | Me | NMe | O | Me | OMe |
| 118 | H | H | NEt | O | Me | OMe |
| 119 | H | Me | NEt | O | Me | OMe |
| 120 | H | Et | NEt | O | Me | OMe |

TABLE Ic

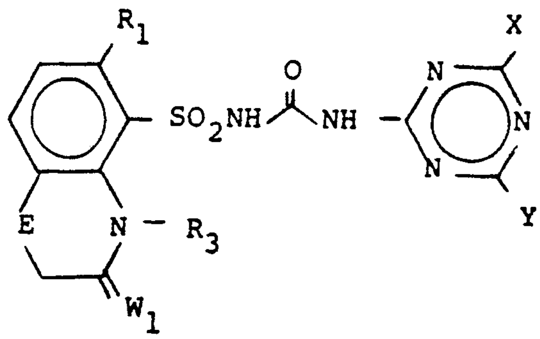

| Compound No. | $R_1$ | $R_3$ | $W_1$ | E | X | Y |
|---|---|---|---|---|---|---|
| 121 | H | H | O | O | Me | OMe |
| 122 | H | H | O | O | OMe | OMe |
| 123 | H | Me | O | O | Me | OMe |
| 124 | H | Me | O | O | OMe | OMe |
| 125 | H | Me | O | O | OMe | $NMe_2$ |
| 126 | H | Me | O | O | OMe | SMe |
| 127 | H | Me | O | O | OMe | OEt |
| 128 | H | Me | O | O | OEt | NHMe |
| 129 | H | Me | O | O | Me | Me |
| 130 | H | Me | S | O | Me | OMe |
| 131 | H | Me | S | O | OMe | OMe |
| 132 | H | Et | O | O | Me | OMe |
| 133 | H | Et | O | O | OMe | OMe |
| 134 | H | Me | O | S | Me | OMe |
| 135 | H | Me | O | S | OMe | OMe |
| 136 | H | Me | S | S | Me | OMe |
| 137 | H | Me | O | SO | Me | OMe |
| 138 | H | Me | O | SO | OMe | OMe |
| 139 | H | Me | O | SO | Me | OMe |
| 140 | H | Et | O | SO | Me | OMe |
| 141 | H | Me | O | $SO_2$ | Me | OMe |
| 142 | H | Me | O | $SO_2$ | OMe | OMe |
| 143 | H | Me | S | $SO_2$ | Me | OMe |
| 144 | H | Et | O | $SO_2$ | Me | OMe |

TABLE 1c (Continued)

| Compound No. | $R_1$ | $R_3$ | $W_1$ | E | X | Y |
|---|---|---|---|---|---|---|
| 145 | H | H | O | NH | Me | OMe |
| 146 | H | H | O | NH | OMe | OMe |
| 147 | H | Me | O | NH | Me | OMe |
| 148 | H | Me | O | NH | OMe | OMe |
| 149 | H | Me | S | NH | Me | OMe |
| 150 | H | H | O | NMe | Me | OMe |
| 151 | H | H | O | NMe | OMe | OMe |
| 152 | H | Me | O | NMe | Me | OMe |
| 153 | H | Me | O | NMe | OMe | OMe |
| 154 | H | Me | S | NMe | Me | OMe |
| 155 | Cl | Me | O | O | Me | OMe |
| 156 | Cl | Me | O | S | Me | OMe |
| 157 | Cl | Me | O | NMe | Me | OMe |
| 158 | Me | Me | O | O | Me | OMe |
| 159 | Me | Me | O | S | Me | OMe |
| 160 | Me | Me | O | NMe | Me | OMe |
| 161 | OMe | Me | O | O | Me | OMe |
| 162 | OMe | Me | O | S | Me | OMe |
| 163 | OMe | Me | O | NMe | Me | OMe |
| 164 | $CO_2Me$ | Me | O | O | Me | OMe |
| 165 | $CO_2Me$ | Me | O | S | Me | OMe |
| 166 | $CO_2Me$ | Me | O | NMe | Me | OMe |
| 167 | $CONMe_2$ | Me | O | O | Me | OMe |
| 168 | $CONMe_2$ | Me | O | S | Me | OMe |
| 169 | $CONMe_2$ | Me | O | NMe | Me | OMe |

TABLE 1d

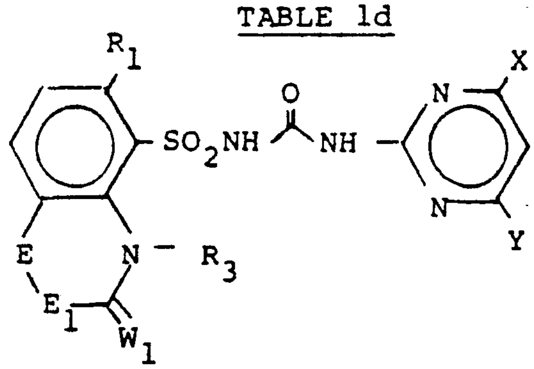

| Compound No. | $R_1$ | $R_3$ | $W_1$ | $E_1$ | E | X | Y |
|---|---|---|---|---|---|---|---|
| 170 | H | H | O | CHMe | O | Me | OMe |
| 171 | H | Me | O | CHMe | O | Me | OMe |
| 172 | H | Me | O | CHMe | O | OMe | OMe |
| 173 | H | Me | O | CHMe | O | Cl | OMe |
| 174 | H | Me | O | CHMe | O | $OCF_2H$ | $OCF_2H$ |
| 175 | H | Me | S | CHMe | O | Me | OMe |
| 176 | H | Me | S | CHMe | O | OMe | OMe |
| 177 | H | Et | O | CHMe | O | Me | OMe |
| 178 | H | Et | S | CHMe | O | Me | OMe |
| 179 | H | Me | O | CHMe | S | Me | OMe |
| 180 | H | Et | O | CHMe | S | Me | OMe |
| 181 | H | Me | O | CHMe | SO | Me | OMe |
| 182 | H | Et | O | CHMe | SO | Me | OMe |
| 183 | H | Me | O | CHMe | $SO_2$ | Me | OMe |
| 184 | H | Et | O | CHMe | $SO_2$ | Me | OMe |
| 185 | H | Me | O | CHMe | NH | Me | OMe |
| 186 | H | Me | O | CHMe | NMe | Me | OMe |
| 187 | H | Et | O | CHMe | NMe | Me | OMe |
| 188 | H | Me | O | CHEt | O | Me | OMe |
| 189 | H | Et | O | CHEt | O | Me | OMe |
| 190 | H | Me | O | CHPr | O | Me | OMe |
| 191 | H | Me | O | CHBu | O | Me | OMe |
| 192 | H | Me | O | CHPh | O | Me | OMe |
| 193 | H | Me | O | CHez | O | Me | OMe |

TABLE 1d (Continued)

| Compound No. | $R_1$ | $R_2$ | $W_1$ | $E_1$ | E | X | Y |
|---|---|---|---|---|---|---|---|
| 194 | Cl | Me | O | CHMe | O | Me | OMe |
| 195 | $CF_3$ | Me | O | CHMe | O | Me | OMe |
| 196 | Me | Me | O | CHMe | O | Me | OMe |
| 197 | OMe | Me | O | CHMe | O | Me | OMe |
| 198 | $CO_2Me$ | Me | O | CHMe | O | Me | OMe |
| 199 | $CONMe_2$ | Me | O | CHMe | O | Me | OMe |
| 200 | H | H | O | $(CH_2)_2$ | O | Me | OMe |
| 201 | H | Me | O | $(CH_2)_2$ | O | Me | OMe |
| 202 | H | Me | O | $(CH_2)_2$ | O | OMe | OMe |
| 203 | H | Me | O | $(CH_2)_2$ | O | Cl | OMe |
| 204 | H | Me | O | $(CH_2)_2$ | O | $OCF_2H$ | $OCF_2H$ |
| 205 | H | Me | S | $(CH_2)_2$ | O | Me | OMe |
| 206 | H | Et | O | $(CH_2)_2$ | O | Me | OMe |
| 207 | H | Et | S | $(CH_2)_2$ | O | Me | OMe |
| 208 | H | Me | O | $(CH_2)_2$ | S | Me | OMe |
| 209 | H | Me | O | $(CH_2)_2$ | SO | Me | OMe |
| 210 | H | Me | O | $(CH_2)_2$ | $SO_2$ | Me | OMe |
| 211 | H | Me | O | $(CH_2)_2$ | NH | Me | OMe |
| 212 | H | Me | O | $(CH_2)_2$ | NMe | Me | OMe |
| 213 | Cl | Me | O | $(CH_2)_2$ | O | Me | OMe |
| 214 | $CF_3$ | Me | O | $(CH_2)_2$ | O | Me | OMe |
| 215 | Me | Me | O | $(CH_2)_2$ | O | Me | OMe |
| 216 | OMe | Me | O | $(CH_2)_2$ | O | Me | OMe |
| 217 | $CO_2Me$ | Me | O | $(CH_2)_2$ | O | Me | OMe |
| 218 | $CONMe_2$ | Me | O | $(CH_2)_2$ | O | Me | OMe |

The compounds of the invention may be prepared by a variety of methods and in a further aspect the invention provides methods for the preparation of compounds of formula I.

Conveniently the preparation of the compounds of the invention can be considered in three parts.

Part A involves the preparation of fused phenylsulfonamides of the formula II

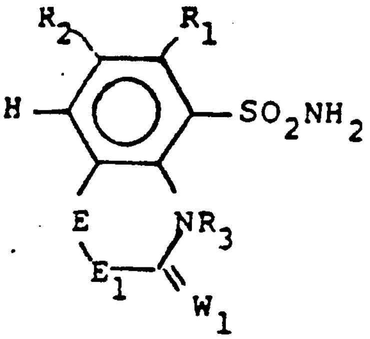

II

wherein $R_1$, $R_2$, $R_3$, $W_1$, E and $E_1$ are as defined for formula I. The sulfonamides of formula II can be prepared in a variety of ways including the general methods outlined below.

(a) The preparation of aromatic sulfonamides from the corresponding anilines by diazotization and reaction of the diazonium salt with sulfur dioxide is well known in the art (J. Org Chem., 25, 1824 (1960).

$$ArNH_2 \xrightarrow[2)\ SO_2,\ CuCl]{1)\ NaNO_2/HCl} ArSO_2Cl \xrightarrow{NH_3} ArSO_2NH_2$$

Some of the necessary amino-benzo-azinones III have been described in the literature (see for example "Rodds Chemistry of carbon compounds", Vol. IV H, p 463, 1978) and they may be prepared for example by reduction of the corresponding nitro compounds.

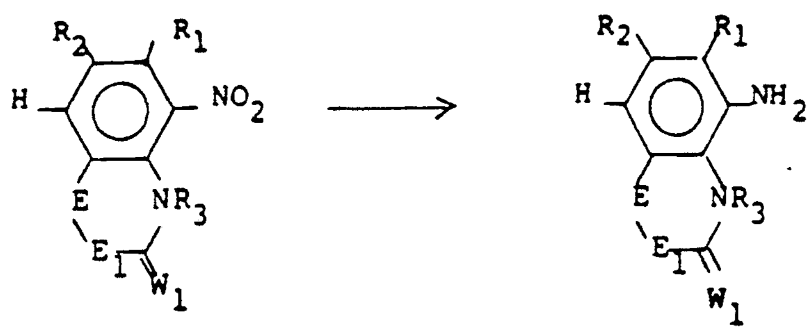

(b) The bicyclic aromatic sulfonamides II may also be prepared from suitable anilines utilizing ortho lithiation of N-pivaloylanilines. Such ortho functionalization of aromatic amines is well documented in the art [see e.g., J. Org. Chem., 44, 1133 (1979)]. The intermediate sulfinates IV are converted by standard procedures to sulfonamides V.

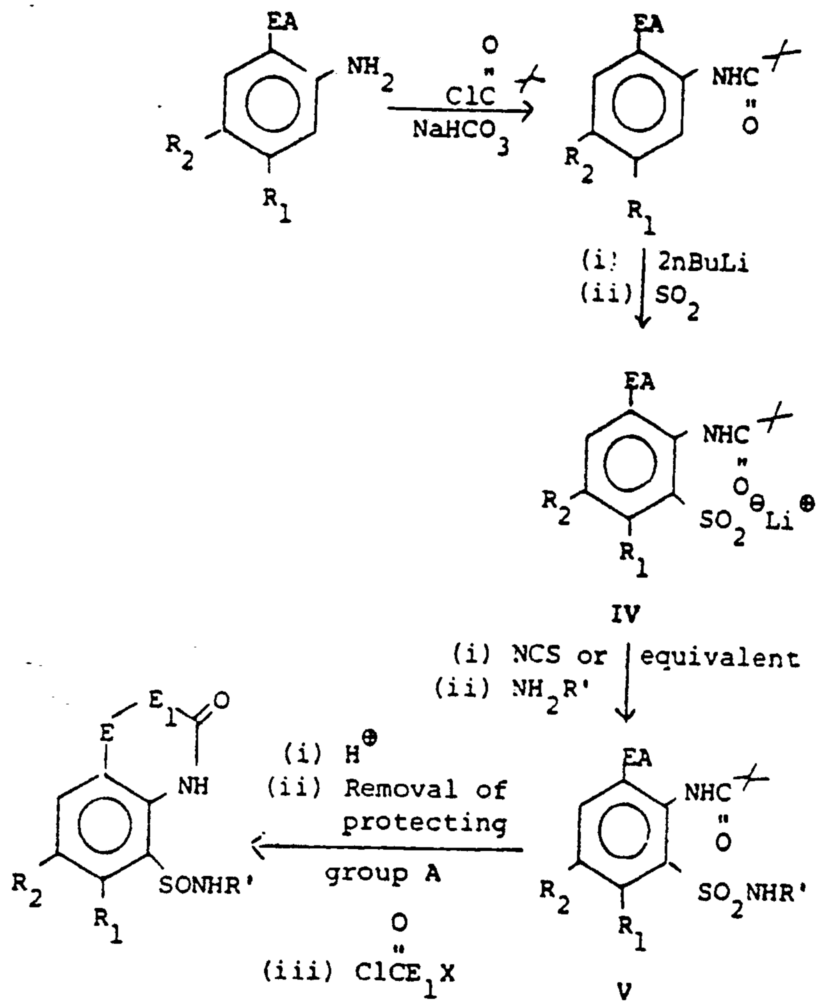


(c) The insertion of sulfur ortho to nitrogen may also be achieved by sulfurization procedures. One such route involves the oxidative cyclization of thiourea derivatives of anilines followed by cleavage of the benzothiazole intermediates VI. Such preparations are well documented in the literature [see e.g. Synthetic Communications, 10, 167(1980) and Heterocycles, 22, 87 (1984)].

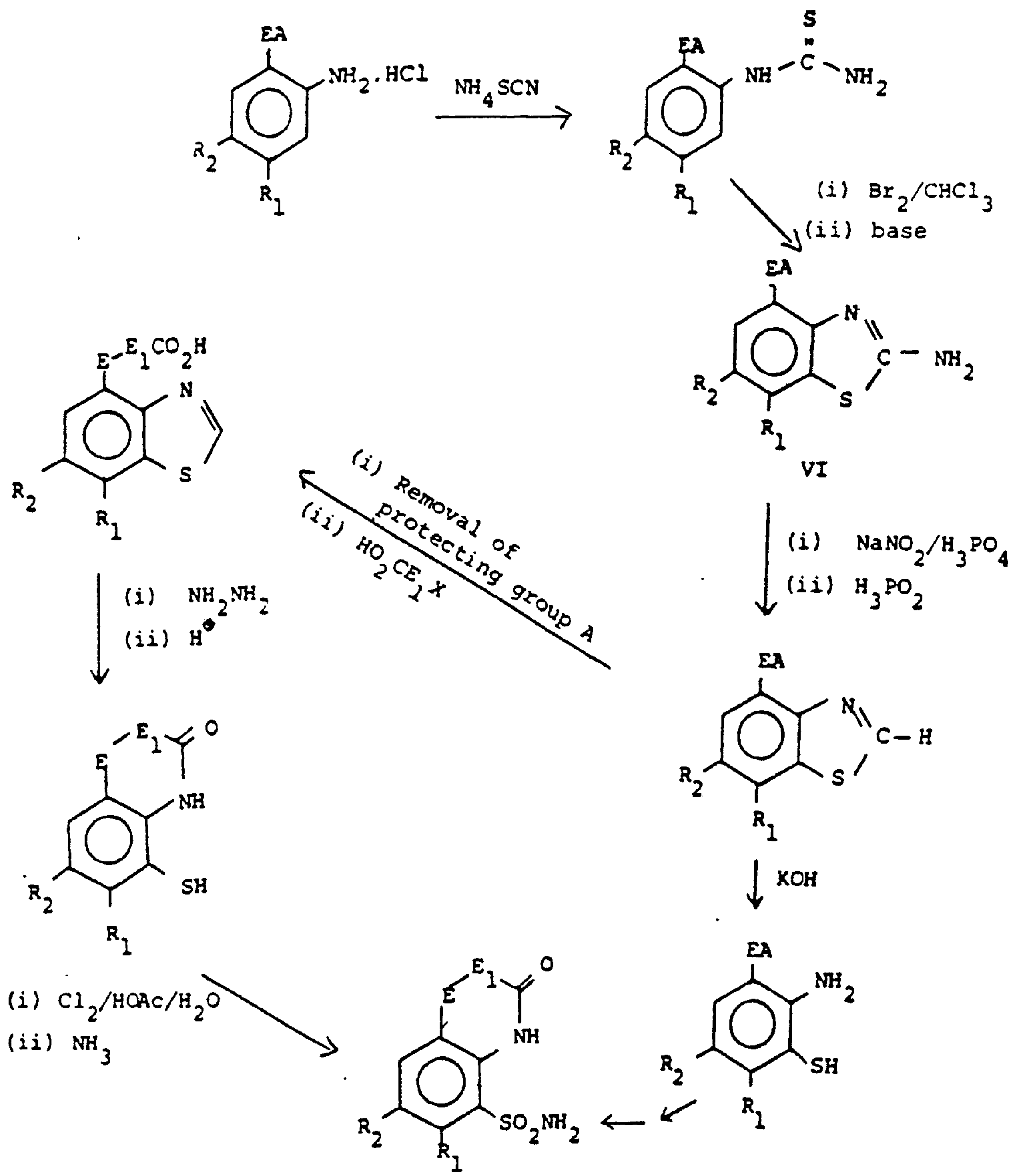


Alternatively, introduction of a thiol group ortho to the nitrogen of a suitable aniline or derivative may be achieved by the Herz reaction, [see e.g., Chem. Rev., 57 1011 (1957)]. In the procedure anilines or derivatives are condensed with sulfur monochloride, and the resultant 1,3,2-benzothiazathiolium chlorides VII hydrolyzed to give the o-aminoaryl thiols. The latter are converted by standard procedures to the sulfonamides.

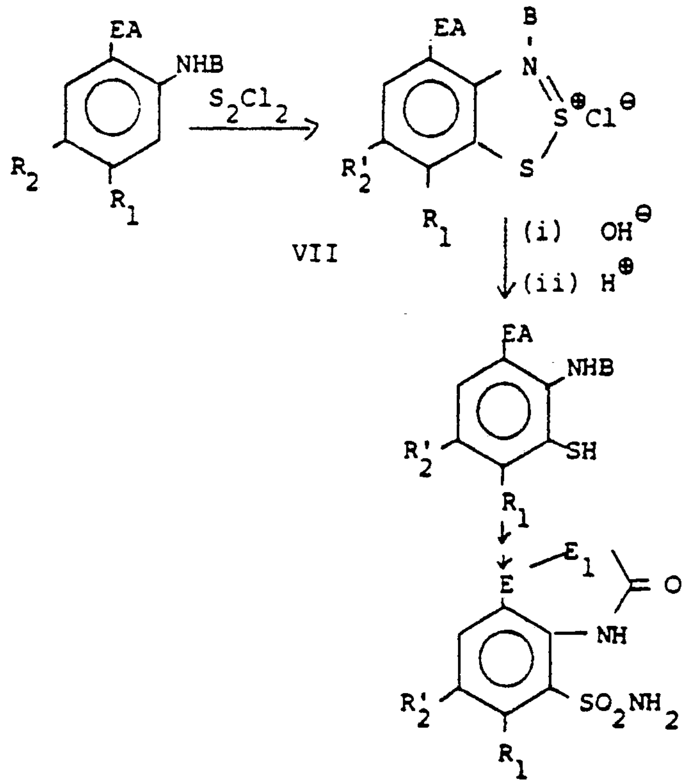


VII

(d) Another route to the sulfonamides II (E = O) involves nitration of resorcinols, followed by reduction and subsequent cyclization to give the phenols VIII. Conversion to thiocarbamates, followed by pyrolysis then affords thiols, which can be converted to sulfonamides by standard procedures.

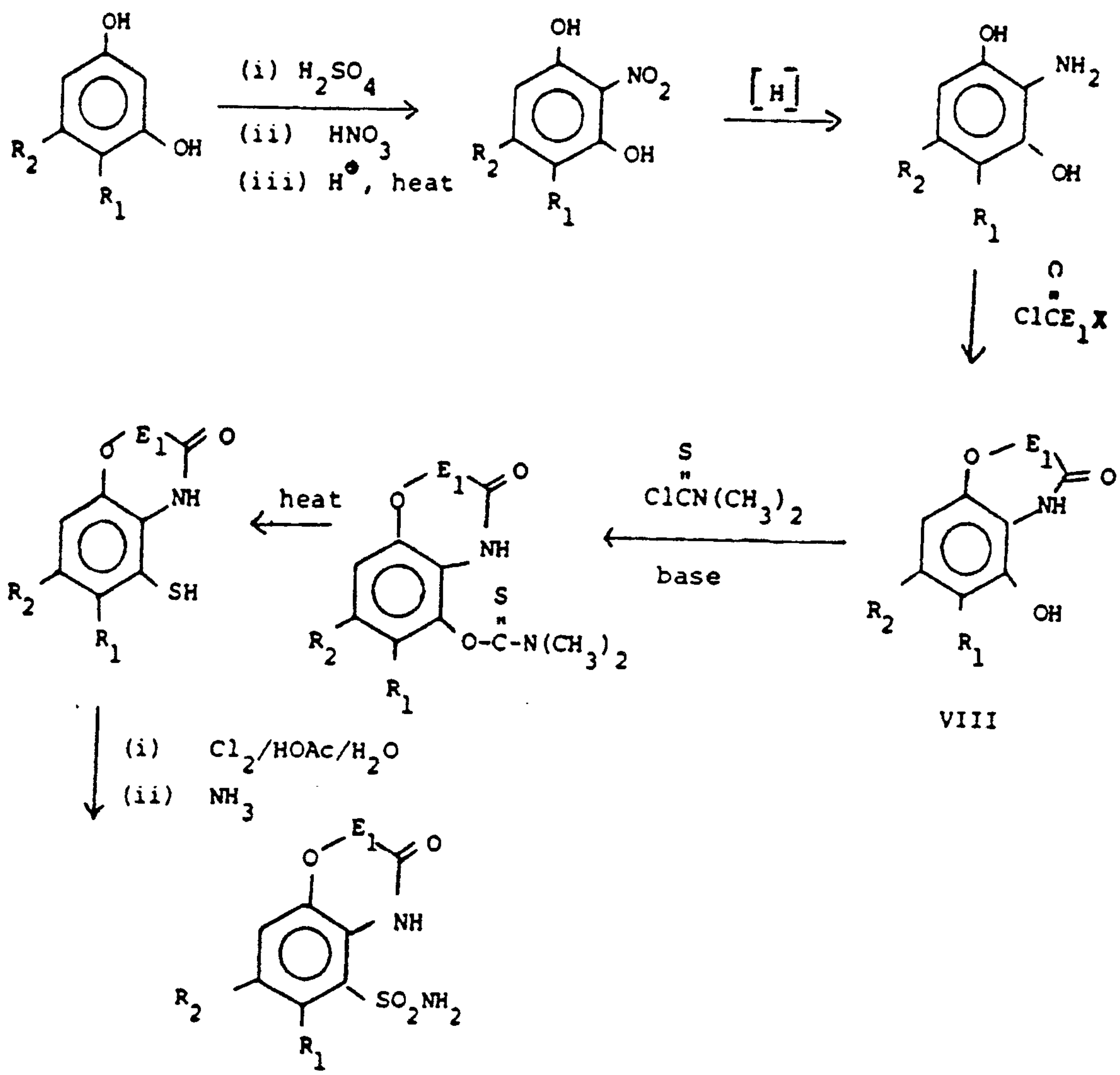


VIII

(e) Preparation of compounds of formula II in which $R_3$ is not hydrogen can be carried out by alkylation of the nitrogen using standard techniques.

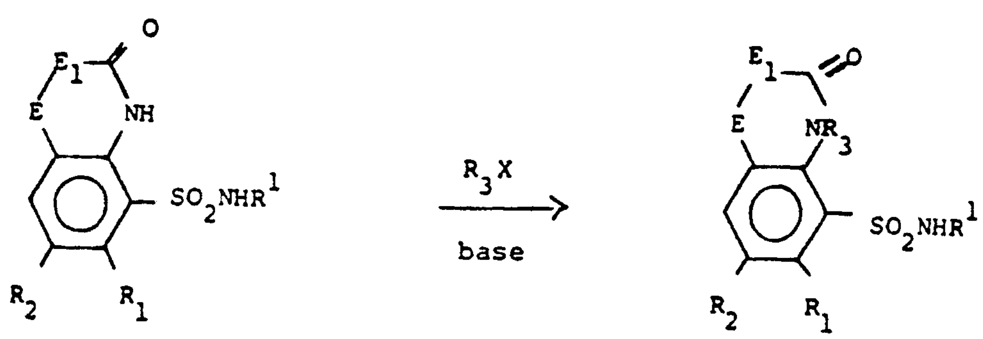


IIa

IIb

Alternatively, alkylation of the nitrogen can be accomplished at an earlier stage of the reaction sequence e.g.,

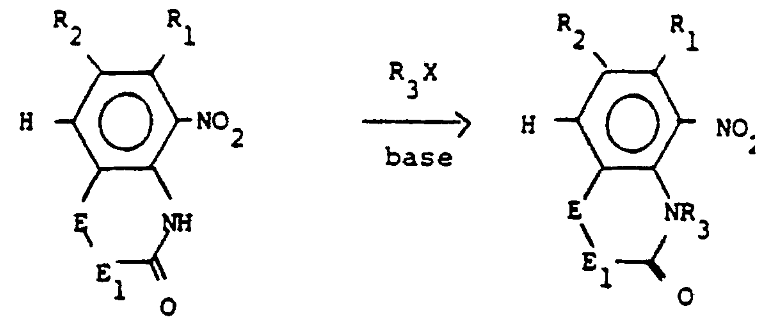


IIIc IIId

(f) Compounds of formula II in which $W_1$ is sulfur may be prepared from the corresponding oxygen compounds by treatment with reagents such as phosphorous pentasulfide and Lawesson's reagent.

In parts (b) and (c) above, group A is a suitable protecting group for the function -EH. In part (c) group B is a suitable protecting group for the anilino NH: in this part groups A and B may together form the linking bridge $-E_1$-C(O)- between the nitrogen N and group E. In parts (b) and (e) group R′ is either hyrogen or a tertiary butyl group: the latter protecting group can be removed readily with e.g., trifluoroacetic acid. In part (c), the nature of $R_2$ will depend upon the nature of $R_2$: replacement of $R_2$ by chlorine may occur. Such a chlorine atom may be hydrogenolyzed out if so desired. In each of parts (b), (c), (d) and (e) above, substituent X is a leaving group, preferably a halogen atom.

Part B of the preparation of the compounds of the invention involves the preparation of the various nitrogen-containing heterocycles A-1 to A-7.

The heterocyclic amines of Formula IX can be prepared by methods known in the literature, or simple modifications thereof, by one skilled in the art.

HN -A IX
 |
 R

For a review of the synthesis and reactions of 2- amino- and 2-methylaminopyrimidines (IX, A = A-1, Z = CH) see The Chemistry of Heterocyclic Compounds, Vol. 16, Wiley-Interscience, New York (1962). For a review of the synthesis and reactions of 2-amino- and 2-methylamino-s-triazines (IX, A = A-1, Z = N) see The Chemistry of Heterocyclic Compounds, Vol. 13, Wiley-Interscience, New York (1959), and F. C. Schaefer and K. A. Huffman, J. Org. Chem., 28, 1812 (1963). EP-A No. 84,224 and W. Braker et al., J. Amer. Chem. Soc., 69, 3072 (1947) describe methods for preparing aminopyrimidines and triazines substituted by acetal groups such as dialkoxymethyl or 1,3-dioxolan-2-yl. U.S. Patent 4,515,626 describes methods for the synthesis of cyclopropyl-pyrimidines and triazines substituted by such groups as alkyl, haloalkyl, alkoxy, haloalkoxy, alkylamino, dialkylamino or alkoxyalkyl.

The 5,6-dihydrofuro[2.3-d]pyrimidine-2-amines, the cyclopenta[d]pyrimidines-2-amines (formula IX wherein A is A-2) and the 6,7-dihydro-5H-pyrano[2,3-d]pyrimidin-2-amines (formula IX wherein A is A-3) can be prepared as taught in U.S. 4,339,267. The furo[2,3-d]pyrimidin-2-amines (IX, A is A-4) are described in U.S. 4.487,626.

Compounds of formula IX where A is A-5, are described in EP-A-73,562. Compounds of Formula IX where A is A-6, are described in US 4,496,392.

The amines of Formula IX where A is A-7 can be prepared by methods taught in European Publication No. 125,864 (published 21/11/84) or by suitable modifications that would be obvious to one skilled in the art.

Part C of the preparation of the compounds of the invention (formula I) involves the coupling of the sulfonamides of formula II with the heterocyclic amines of formula IX. The compounds of formula I can be prepared by one or more of the methods described below.

a) Many of the compounds of formula I can be prepared by reacting a sulfonylisocyanate or a sulfonylisothiocyanate of formula X with a heterocyclic amine of formula IX.

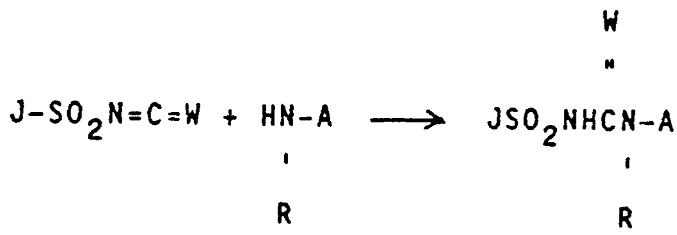

X IX I

where J represents the bicyclic system

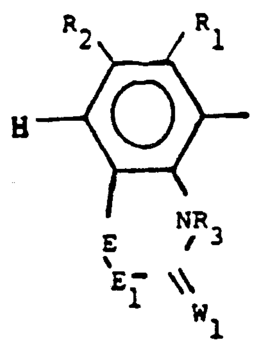

and W, A and R are as previously defined.

The reaction is carried out at 25° to 100°C in an inert, aprotic solvent such as methylene chloride or xylene for 0.5 to 24 hours as taught in U.S. Patent 4,127,405

The intermediate sulfonylisocyanates (formula X wherein W = O) and isothiocyanates (formula X wherein W = S) may be prepared by a variety of methods which are well known in the art and are described for example in European Patent Application 212,779 and the references cited therein.

b) Many of the compounds of formula I, where W is oxygen, can be prepared by reacting a phenyl carbamate of formula XI with a suitable amine of formula IX.

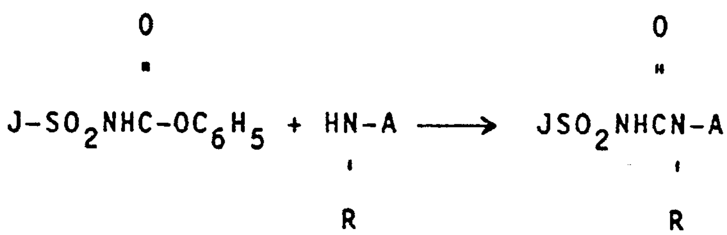

XI IX Ia

The reaction is carried out at 0° to 100°C in a solvent such as dioxane or N,N-dimethyformamide for 0.5 to 24 hours. The required carbamates XI are prepared by reacting the corresponding sulfonamides II with diphenylcarbonate in the presence of a strong base.

c) Compounds of formula Ia can also be made by reacting a heterocyclic carbamate of formula XII with a suitable sulfonamide of formula II.

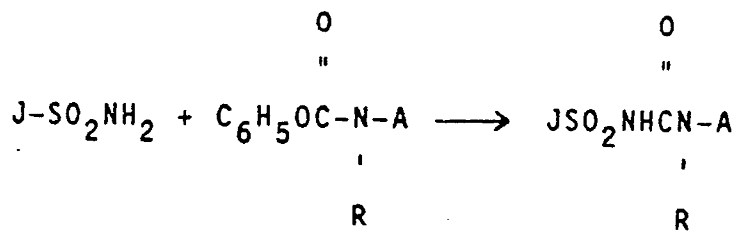

II XII Ia

The reaction is carried out at 0° to 100°C in a solvent such as acetonitrile, dioxane or N,N-dimethylformamide in the presence of a non-nucleophilic base such as DBU for 0.2 to 24 hours. The required phenylcarbamates XII are prepared by reacting the corresponding heterocyclic amines IX with diphenylcarbonate or phenylchloroformate in the presence of a strong base.
Alternatively, the methylcarbamate derivative of the heterocyclic amine IX may be used.

d) Some of the compounds of the invention of formula Ib can be prepared by reacting a sulfonamide II with a heterocyclic isocyanate on isothiocyanate of formula XIII.

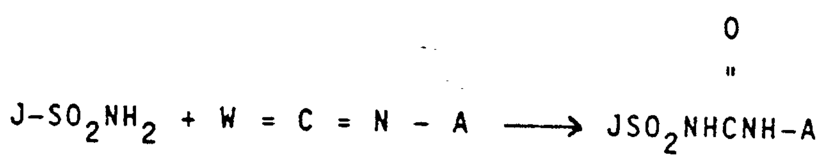

II XIII Ib

The reaction is carried out at 25° to 80°C in an inert, aprotic solvent such as acetone or acetonitrile in the presence of a base such as potassium carbonate for 0.5 to 24 hours. The required heterocyclic isocyanates and iso-thiocyanates XIII are prepared from the corresponding amines $H_2NA$ which would be known to one skilled in the art as taught in EPO Publication 35,893.

In each of parts b), c) and d) above the groups J, W, A and R are as previously described.

Certain of the intermediate compounds of formulae II, III IV, V, VI, VII and VIII are novel compounds and therefore in further embodiments the invention provides novel compounds of formulae II, III, IV, V, VI, VII and VIII and processes for the preparation thereof.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g. hydroxide, alkoxide, carbonate or hydroxide). Quaternary amine salts can be made by similar techniques.

Salts of compounds of Formula I can also be prepared by exchange of one cation to another. Cationic exchange can be effected by direct treatment of an aqueous solution of a salt of a compound of Formula I, (e.g, alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I, (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I, with a suitable acid, e.g., p-toluenesulfonic acid, trichloroacetic acid or the like.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

The compounds of formula I may be applied directly to the plant (post-emergence application) or to the

soil before the emergence of the plant (pre-emergence application).

The compounds of formula I may be used on their own to regulate the growth of plants but in general are preferably used in the form of a composition comprising a compound of the invention in admixture with a carrier comprising a solid or liquid diluent. Therefore, in a still further aspect the invention provides plant growth regulating compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 1 ppm to 2% of active ingredient, while concentrated compositions may contain from 20 to 99% of active ingredient, although from 20 to 70% is usually preferred.

The solid compositions may be in the form of granules, or dusting powders wherein the active ingredient is mixed with a finely divided solid diluent such as kaolin, bentonite, kieselguhr, dolomite, calcium carbonate, talc, powdered magnesia, Fuller's earth or gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion or an active ingredient in water optionally containing a surface-active agent, or may comprise a solution or dispersion of an active ingredient in a water-immiscible organic solvent which is dispersed as droplets in water.

The rate of application of the compounds of the invention will depend on a number of factors including, for example, the compound chosen for use, the identity of the plants to be treated, the formulations selected for use and whether the compound is to be applied for foliage or root uptake. As a general guide, however, an application rate of from 0.001 to 10 kilograms, per hectare is suitable while from 0.01 to 5 kilograms per hectare may be preferred.

The compositions of the invention may comprise, in addition to one or more compounds of the invention, one or more compounds not of the invention but which possess biological activity. In certain applications it may be preferred to use a herbicidal composition comprising a mixture of at least one herbicidal compound of formula I as hereinbefore defined and at least one other herbicide.

The compounds of this invention and their preparation are further illustrated by the following examples.

## Example 1

### N-[(4-Methoxy-6-methylpyrimidin-2-yl)aminocarbonyl]-4-methyl-2H-1,4-benzoxazin-3(4H)-one-5-sulfonamide (6)

#### (i) 5-Nitro-2H-1,4-benzoxazin-3(4H)-one

This compound was made from 2-amino-3-nitrophenol according to a modification of the general procedure described inOrg. Prep. Proced. Int., 14, 195(1982).

2-amino-3-nitrophenol (25.5 g) was added to an ice-chilled, vigorously stirred mixture of isobutyl methyl ketone (100 ml), water (100 ml) and sodium carbonate (21.0 g). Chloroacetyl chloride (16 ml) was then added dropwise with stirring and the cold mixture was allowed to warm to ambient temperature before refluxing for 4 h. The resultant mixture was diluted with aqueous citric acid (10%, 56 ml), stirred an additional 30 min, then filtered. Rinsing of the solid with ethyl acetate afforded the pure benzoxazinone as yellow needles (22.9 g). $^1$H NMR ($CD_3COCD_3$): 4.76 (s, 2H); 7.18 (t, J = 8.2 Hz, 1H); 7.42 (dd, J = 8.1, 1.7 Hz, 1H); 7.90 (dd, J = 8.4, 1.8 Hz, 1H).

#### (ii) 4-Methyl-5-nitro-2H-1,4-benzoxazin-3-(4H)-one

A mixture of 5-nitro-2H-1,4-benzoxazin-3(4H)-one (5.82 g), acetone (100 ml), dimethyl sulfate (10 ml) and anhydrous potassium carbonate (45 g) was refluxed for 1 h, allowed to cool and poured, with swirling, into water (600 ml). Filtration gave the title compound as straw yellow microcrystals (5.79 g). $^1$H NMR ($CD_3COCD_3$): 3.12 (s, 3H); 4.74 (s, 2H); 7.1 - 7.45 (m, 2H); 7.60 (dd, J = 7.2, 2.5 Hz, 1H).

(iii) 5-Amino-4-methyl-2H-1,4-benzoxazin-3(4H)-one

The following reduction is according to a general procedure described in Tet. Lett., 25, 3415(1984).

To a stirred mixture of 4-methyl-5-nitro-2H-1,4-benzoxazin-3(4H)-one (3.31 g) and anhydrous ammonium formate (6.0 g) in dry methanol (45 ml), under nitrogen, was added in one portion at 0°C 10% palladium on charcoal (0.96 g). The mixture was stirred at 0° for 25 min, at ambient temperature for 4 h and then filtered through diatomaceous earth. The filtrate was evaporated in vacuo and the residue partitioned between ethyl acetate and water. After re-extraction of the aqueous layer with ethyl acetate, the combined organic phase was washed successively with water and saturated brine, then filtered through oven-dried cotton wool. Evaporation gave the title amine as a viscous oil which solidified upon cooling to a waxy colourless solid (2.71 g). $^1$H NMR ($CD_3COCD_3$); 3.37 (s, 3H); 4.36 (s, 2H); 4.65 (br s, 2H); 6.3 - 6.85 (m, 3H).

(iv) 4-Methyl-5-sulfonamido-2H-1,4-benzoxazin-3(4H)-one

The modified Sandmeyer reaction is according to the procedure described in J. Org. Chem., 25, 1824-(1960). A stirred mixture of 5-amino-4-methyl-2H-1,4-benzoxazin-3(4H)-one (7.43 g), concd. hydrochloric acid (25 ml) and acetic acid (10 ml) at -5 to 0°C was treated dropwise, with a mixture of sodium nitrite (3.20 g) and water (6 ml).

The stirred diazotized solution was allowed to warm from -5 to 0°C over 45 min and then added to cuprous chloride (1.03 g) in a saturated solution of sulfur dioxide in acetic acid (100 ml) at 0-5°C. Stirring was continued at ambient temperature for 45 min, then a little more cuprous chloride (ca 0.2 g) added and 15 min later the reaction mixture was poured into ice-water (600 ml) and the product extracted with ethyl acetate (thrice). The organic solution was washed successively with water (six times) and saturated brine, then filtered through oven-dried cotton wool and evaporated in vacuo. Excess acetic acid was azeotroped from the residue with acetonitrile, and the crude sulfonyl chloride reacted without further purification. The sulfonyl chloride residue was chilled in ice and treated slowly, with stirring, with concd. aqueous ammonia (33% w/w, 100 ml). After stirring at ambient temperature for 30 min, the mixture was rechilled to 0°C and slowly acidified to pH 4 with concd. hydrochloric acid. Filtration of the mixture gave a first crop of the title sulfonamide as a fawn powder (2.22 g). Extraction of the acidic filtrate with ethyl acetate provided a second crop of product (1.73 g). $^1$H NMR ($CD_3COCD_3$/$d_6$DMSO, 2:3): 3.50 (s, 3H); 4.57 (s, 2H); 7.2 -7.3 (m, 2H); 7.7 - 7.9 (m, 3H).

(v) N-[(4-Methoxy-6-methylpyrimidin-2-yl)aminocarbonyl]-4-methyl-2H-1,4-benzoxazin-3(4H)-one-5-sulfonamide (6)

4-Methyl-5-sulfonamido-2H-1,4-benzoxazin-3(4H)-one (1.16 g) in dry tetrahydrofuran (20 ml) was treated dropwise with 1,8-diazabicyclo[5.4.0]undec-7-ene (1.05 ml), and the resultant solution stirred at ambient temperature for 30 min. Phenyl (4-methoxy-6-methyl-2-pyrimidinyl)carbamate (1.36 g) was then added and stirring continued for 4.25 h, whereupon the reaction mixture was poured into ice-cold aqueous citric acid (10%, 150 ml). This mixture was extracted with ethyl acetate (twice), and the organic extracts washed successively with aqueous citric acid (10%) containing a little brine (twice), water and saturated brine, followed by filtration through dry cotton wool and evaporation. The residue was warmed in ether, and after cooling the solid was filtered, triturated with several portions of ether and refiltered to give the title sulfonyl urea as an off-white powder (1.19 g). $^1$H NMR ( $d_6$ DMSO): 2.39 (s, 3H); 3.47 (s, 3H); 3.93 (s, 3H); 4.60 (s, 2H); 6.59 (s, 1H); 7.15 - 7.45 (m, 2H); 7.87 (dd, J = 7.6, 2.2 Hz; 1H); 10.84 (s, 1H); 13.97 (br s, 1H).

Example 2

N-[(4,6-Dimethoxypyrimidin-2-yl)aminocarbonyl]-4-methyl-2H-1,4-benzoxazin-3(4H)-one-5-sulfonamide (7)

4-Methyl-5-sulfonamido-2H-1,4-benzoxazin-3(4H)-one [0.72g, from Example 1(v)] in dry N,N-dimethylformamide (5 ml) at 10°C was treated dropwise with 1,8-diazabicyclo[5.4.0]undec-7-ene(0.67 ml) and the resultant solution stirred at 0°C for 5 min before the addition of phenyl (4,6-dimethoxypyrimidinyl)carbamate

(0.70 g). Stirring was continued at 0-10°C for 1 h, then at ambient temperature for 1 hr, whereupon the reaction mixture was poured into ice-cold aqueous citric acid (10%, 50 ml). This mixture was filtered, and the precipitated solid rinsed successively with aqueous citric acid (twice) and water, then drained and dried over phosphorus pentoxide to give the title sulfonylurea as a fawn powder (1.21 g). $^1$H NMR ($d_6$DMSO); 3.92(s, 6H); 4.76(s, 2H); 6.00(s, 1H); 7.1-7.7(m, 3H); 9.65(s, 1H); 10.72(s, 1H).

Example 3

N-[(4-Methoxy-6-methylpyrimidin-2-yl)aminocarbonyl]-4-methyl-2H-1,4-benzoxazin-3(4H)-thione-5-sulfonamide (19)

(i) 4-methyl-5-sulfonamido-2H-1,4-benzoxazin-3(4H)-thione

A mixture of 4-methyl-5-sulfonamido-2H-1,4-benzoxazin-3(4H)-one [1.48g, from Example 1 (iv)] and phosphons pentasulfide (2.04g) in pyridine (15 ml) was warmed at 80-100°C for 3.5h. The mixture was allowed to cool to ambient temperature, ethyl acetate was added and the mixture was washed with aqueous citric acid (10%, 5 x 100 ml). The dried organic layer was filtered through a short column of silica and evaporated to leave the title thioamide (0.82 g) as a yellow solid. $^1$H NMR ($CD_3COCD_3$): 3.94(S, 3H); 4.81-(s,2H); 7.1-7.4(m,2H); 7.84 (dd, J=6.2, 3.1 Hz; 1H). This product was chromatographed [$SiO_2$, ethyl acetate: petroleum ether b.p. 60-80°C, 1:3] before synthesis of sulfonylureas.

(ii) N-(4-methoxy-6-methylpyrimidin-2-yl)amino carbonyl]-4-methyl-2H-1,4-benzoxazin-3(4H)-thione-5-sulfonimide (19)

A solution of the above sulfonamide (0.34g) and phenyl (4-methoxy-6-methyl-2-pyrimidinyl) carbamate (0.32g) in dry tetrahydrofuran (10 ml) was treated dropwise at 0°C with 1,8-diazabicyclo[5.4.0]undec-7-ene (0.25 ml), and the resultant solution stirred at ambient temperature for 18 h. After pouring into ice-cold aqueous citric acid (10%, 75 ml) the mixture was extracted with ethyl acetate (twice) and the organic extracts washed as in Example 1 (v). Removal of the solvent and trituration of the residue with ether furnished the title sulfonylurea (0.28 g). $^1$H NMR ($CD_3COCD_3$/$d_6$ DMSO, 2:1): 2.45(s,3H); 3.94(s,3H); 3.97 (s,3H); 4.85(s, 2H); 6.55(s,1H); 7.3-7.45 (m, 2H); 7.97 (pseudot, J = 5.1$H_z$, 1H); 10.64 (brs, 1H); 14.04 (vbrs, 1H).

Example 4

N-[(4-methoxy-6-methylpyrimidin-2yl)aminocarbonyl-4-ethyl-2H-1,4-benzoxazin-3-(4H)-one-5-sulfonamide (23)

This was prepared according to the general procedure described in Example 1. Compound No. 23 (1.18 g) was an off-white solid. $^1$H NMR ($d_6$DMSO): 0.84(6, J=7.1$H_3$, 3H); 2.40(s,3H); 3.94(s,3H); 4.28(q, J=6.9Hz, 2H); 4.49(s, 2H); 6.61(s,1H; 7.2-7.45(m,2H): 7.86(dd, J=7.1, 2.7Hz, 1H); 10.92(brs, 1H); 13.93-(vbrs, 1H).

Example 5

N[(4-methoxy-6-methylpyrimidin-2yl)aminocarbonyl-4-ethyl-2H-1,4-benzoxazin-3(4H)-thione-5-sulfonamide (27)

$^1$H NMR ($d_6$ DMSO): 0.88 (t, J=7.2Hz,3H); 2.40 (s,3H); 3.90(s,3H); 4.65(s,2H); 4.89(m,3H); 6.59(s,1H); 7.32(m,2H); 7.80(m,1H); 10.94(brs, 1H); 13.47 (vbrs, 1H).

Example 6

N-[(4-methoxy-6-methylpyrimidin-2-yl)aminocarbonyl]-4-propyl-2H-1,4-benzoxazin-3(4H)-one-5-sulfonamide (31)

Compound No. 31 was prepared by coupling 4-propyl-5-sulfonamido-2H-1,4-benzoxazin-3(4H)-one (0.23 g) with methyl (4-methoxy-6-methyl-2-pyrimidinyl)carbomate (0.20 g) according to the procedure described in Example 1(V). The title compound (0.18g) as a white solid. $^1$H NMR ($CD_3COCD_3$/$d_6$DMSO, 1:1): 0.63 (t, J=7.3Hz, 3H); 1.31(m,2H); 2.46(s,3H); 3.97(s,3H); 4.29(t, J= 6.8Hz,2H); 4.54(s,2H); 6.55(s,1H); 72-7.5-(m,2H); 7.95(dd, J=6.8, 2.8Hz, 1H); 10.60(brs, 1H); 14.01(vbrs, 1H).

Example 7

N-[4,6-Dimethoxypyrimidin-2-yl)aminocarbonyl]-4-benzyl-2H-1,4-benzoxazin-3(4H)-one-5-sulfonamide (34)

Compound No. 34 was prepared by coupling 4-benzyl-5-sulfonamido-2H-1,4-benzoxazin-3(4H)-one (0.50g) with phenyl (4,6-dimethoxy-2-pyrimidinyl)carbamate (0.48 g) according to the procedure described in Example 1 part (v). The title sulfonylurea (0.52 g) was a white solid. $^1$H NMR ($CD_3COCD_3$): 3.99(s,6H); 4.55(s,2H); 5.48(s,2H); 5.92(s,1H); 7.07(m,2H); 7.20(m,3H); 7.30(t, J=7.9Hz, 1H); 7.37(dd, J=8.0, 1.9Hz, 1H); 7.93 (dd, J=7.7, 1.9Hz, 1H); 10.08(brs, 1H); 13.18 (vbrs, 1H).

Example 8

N-[(4-Methoxy-6-methylpyrimidin-2-yl)aminocarbonyl]-2,4-dimethyl-2H-1,4-benzoxazin-3(4 H)-one-5-sulfonamide (171)

(i) 2-Methyl-5-nitro-2H-1,4-benzoxazin-3(4 H)-one

To an ice-chilled, vigorously stirred, mixture of 2-amino-3-nitrophenol(11.4 g), isobutyl methyl ketone (50 ml), water (50 ml) and sodium bicarbonate (15.6 g) was added dropwise 2-bromopropionyl bromide (9.4 ml). The resultant mixture was allowed to warm to ambient temperature, stirred and refluxed for 1.75 h, stood for 3 days, then filtered. Rinsing of the solid with several portions of ethyl acetate gave the title benzoxazinone as yellow needles (11.7 g). $^1$H NMR ($CD_3COCD_3$/$d_6$ DMSO 3.1) : 1.52(d, J=6.8 Hz, 3H); 4.86 (q, J=6.8Hz,1H); 7.18(t, J=8.1Hz,1H); 7.44 (dd, J=8.1, 1.7Hz, 1H); 7.84 (dd, J=8.3, 1.7 Hz, 1H).

(ii) 2,4-Dimethyl-5-nitro-2H-1,4-benzoxazin-3(4H)-one

A mixture of 2-methyl-5-nitro-2H-1,4-benzoxazin- 3(4H)-one (6.34 g), anhydrous potassium carbonate (45 g), dimethyl sulfate (10 ml) and acetone (100 ml) was stirred and refluxed for 4 h, then allowed to cool. The mixture was poured, with swirling, into water (600 ml); and filtration gave the title compound as a straw yellow powder (6.16 g). $^1$H NMR ($CD_3COCD_3$): 1.55 (d, J=6.8Hz, 3H); 3.12(s,3H); 4.75 (q, J=6.5Hz, 1H); 7.1-7.5 (m,2H); 7.61(dd, J=7.3, 2.6 Hz, 1H).

(iii) 5-Amino-2,4-dimethyl-2H-1,4-benzoxazin-3(4H)-one

2,4-Dimethyl-5-nitro-2H-1,4-benzoxazin-3(4H)-one (5.75 g) was reduced using ammonium formate as a catalytic hydrogen transfer agent via the method described in Example 1, Part (iii). The title aniline was obtained as an off-white solid (4.63 g). $^1$H NMR ($CD_3COCD_3$): 1.43 (d, J=6.8Hz, 3H); 3.36(s,3H); 4.30 (q, J=6.7Hz, 1H); 4.63(br s, 2H); 6.36 (dd, J=7.7, 1.7Hz, 1H); 6.53 (dd J= 8.2, 1.5 Hz, 1H); 6.81 (t, J= 8.0 Hz, 1H).

(iv) 2,4-Dimethyl-5-sulfonamido-2H-1,4-benzoxazin-3(4 H)-one

The modified Sandmeyer reaction of 5-amino-4-methyl-2H-1,4-benzoxazin-3(4H)-one (4.22 g) was performed by the method described in Example 1, Part (iv). The sulfonyl chloride crystallized upon dilution with ice-water as a light tan solid. $^1H$ NMR ($CDCl_3$): 1.61 (d, J = 6.8Hz, 3H); 3.64 (s,3H); 4.47 (q, J = 6.9Hz, 1H); 7.1-7.5 (m, 2H); 7.86 (dd, J = 7.7, 2.3 Hz, 1H). The crude sulfonyl chloride was suspended in water (10 ml), chilled to 0°C and treated with concd. aqueous ammonia (10 ml). The mixture was stirred at 0°c for 30 min, treated with more ammonia (10 ml), stirred at ambient temperature for 3h then diluted with dichloromethane (10 ml). After an additional 30 min, the reaction mixture was slowly acidified with 5 M hydrochloric acid until pH 4-4.5. The resultant mixture was extracted with ethyl acetate (twice); and the organic extracts washed with water (six times) and saturated brine, then filtered through dry cotton wool and evaporated to give the title sulfonamide as a pale tan solid (3.01 g). $^1H$ NMR ($CD_3COCD_3$/$d_6$DMSO, 1:1): 1.48 (d, J = 6.6 Hz 3H); 3.51(s,3H); 4.49 (q, J = 6.4Hz,1H); 7.2-7.3 (m,2H); 7.69 (br s, 2H); 7.83 (m, 1H).

(v) N[(4-Methoxy-6-methylpyrimidin-2-yl)amino carbonyl]-2,4-dimethyl-2H-1,4-benzoxazin-3(4H)-one-5-sulfonamide (171)

Compound No. 171 was prepared by coupling the above sulfonamide (0.51 g) with methyl (4-methoxy-6-methyl-2-pyrimidinyl)carbamate (0.43 g), according to the method described in Example 6. The title sulfonylurea (0.20 g) precipitated upon acidification of the reaction mixture, as an off-white solid. $^1H$ NMR ($d_6$ DMSO): 1.43 (d, J = 6.6$H_z$,3H); 2.39(s,3H), 3.46(s,3H); 3.93(s,3H); 4.50(q, J = 6.6Hz,1H); 6.59(s,1H); 7.0-7.45(m,2H); 7.45-7.85 (m,1H); 10.85(brs,1H); 13.72(vbrs,1H).

Example 9

Compounds Nos. 1, 2, 5, 20, 24, 28, 32, 36, 123, 132, 172, 175 and 176 were each prepared by coupling the appropriate benzoxazinone sulfonamide and 2-amino heterocycle. The coupling was generally carried out using conditions described in Examples 1-8.

$^1H$ NMR spectral data for each of the sulfonylureas is given in Example 10, Table 2.

Example 10

(Table 2)

TABLE 2

| Compound No. | Solvent | Proton Chemical Shift in ppm |
|---|---|---|
| 1 | $d_6$DMSO | 2.41(s,3H); 3.95(s,3H); 4.73(s,2H); 6.61(s,1H); 7.0-7.3(m,2H); 7.53 (dd, J=7.5, 1.8 Hz, 1H); 9.68(s,1H); 11.06(brs,1H); 13.57(vbrs,1H). |
| 2 | $d_6$DMSO | 3.92(s,6H); 4.76(s,2H); 6.00(s,1H); 7.1-7.45 (m,2H); 7.5-7.7(m,1H); 9.65(s,1H); 10.72(brs,1H); one exchangeable proton not observed. |
| 5 | $d_6$DMSO | 2.42(s,6H); 4.61(s,2H); 7.04(s,1H); 7.15-7.755 (m,2H); 7.90 (dd, J= 7.1, 2.2Hz, 1H); 10.74(s,H); one exchangeable proton not observed. |
| 20 | $CD_3COCD_3$ | 3.94(s,3H); 3.98(s,6H); 4.83(s,2H); 5.92(s,1H); 7.37(m,2H); 7.98(pseudot, J=4.6Hz, 1H); 9.53 (brs,1H); 13.15(brs,1H). |

TABLE 2 (Continued)

| Compound No. | Solvent | Proton Chemical Shift in ppm |
|---|---|---|
| 24 | $d_6DMSO$ | 0.87(t, J=7.3Hz,3H); 3.91 (s,6H); 4.23(q, J= 6.9 Hz, 2H); 4.52(s,2H); 5.99(s,1H); 7.2-7.55 (m,2H);7.85 (dd, J= 7., 2.2Hz, 1H); 10.31 (brs,1H); one exchangeable not observed. |
| 28 | $d_6DMSO$ | 0.99(m,3H); 3.91(s,6H); 4.70(s,2H); 4.78(m,2H); 5.90(s,1H); 7.40(m,2H); 7.85(m,1H); 10.17(brs,H); exchangeable not observed. |
| 32 | $CD_3COCD_3$/ $d_6DMSO$,1:5 | 0.62(t, J= 6.7Hz,3H); 1.35(m,2H); 3.92(3,6H); 4.22(m,2H); 4.56(s,2H); 5.99(s,1H); 7.38(m,2H); 7.92(m,1H); 10.47(brs, 1H); one exchangeable not observed. |
| 36 | $CD_3COCD_3$ | 3.98(s,6H); 4.71(s,2H); 5.88(s,1H); 7.19(d, J= 8.6Hz, 1H); 7.42(d, J=8.6Hz,1H); 10.21 (brs,1H); 13.6(brs,1H). |

TABLE 2 (Continued)

| Compound No. | Solvent | Proton Chemical Shift in ppm |
|---|---|---|
| 123 | $d_6$DMSO | 2.47(s, 3H); 3.45(s, 3H), 3.98(s, 3H); 4.64 (s, 2H); 7.2-7.5(m, 2H); 7.90(dd, J = 7.3, 2.0 Hz, 1H); 11.07 (s, 1H); one exchangeable not observed. |
| 132 | $d_6$DMSO | 0.88(t, J = 6.4 Hz, 3H); 2.47(s, 3H); 3.97(s, 3H); 4.06(q, J = 6.7 Hz, 2H); 4.53(s, 2H); 7.41(m, 2H); 7.87(m, 1H), 11.02(br s, 1H); one exchangeable not observed. |
| 172 | $d_6$DMSO | 1.44(d, J = 6.6 Hz, 3H); 3.91(s, 6H); 4.61(q, J = 6.6Hz, 1H); 6.00(s, 1H), 7.05-7.5(m, 2H); 7.86 (dd, J = 7.3, 2.4 Hz, 1H); 10.51(br s, 1H); one exchangeable not observed. |
| 176 | $CD_3COCD_3$ | 1.51(d, J = 6.7 Hz, 3H); 3.94(s, 9H); 4.82(q, J = 6.7Hz, 1H); 5.91(s, 1H); 6.94(m, 2H); 7.82(m, 1H); 9.31(v br s, 1H); one exchangeable not observed. |

Example 11

This non-limiting Example illustrates the preparation of formulations of the compounds of the invention.

a) Emulsifiable Concentrate

Compound No. 6 was dissolved in toluene/DMSO containing 7% v/v "Teric" N13 and 3% v/v "Kemmat" SC15B to give an emulsifiable concentrate which was diluted with water to the required concentration to give an aqueous emulsion which was applied by spraying.

("Teric" is a Trade Mark and "Teric" N13, is a product of ethoxylation of nonylphenol; "Kemmat" is a Trade Mark and "Kemmat" SC15B is a formulaiton of calcium dodecylbenzenesulfonate).

b) Aqueous Suspension

Compound No. 7 (5 parts by weight) and "Dyapol" PT (1 part by weight) were added to a 2% aqueous solution (94 parts by weight) of "Teric" N8 and the mixture was ball milled to produce a stable aqueous suspension which may be diluted with water to the required concentration to give an aqueous suspension which may be applied by spraying.

("Dyapol" is a Trade Mark and "Dyapol" PT is an anionic suspending agent; "Teric" N8 is a product of ethoxylation of nonylphenol).

c) Emulsifiable Concentrate

Compound No. 19 (10 parts by weight), "Teric" N13 (5 parts by weight) and "Kemmat" SC15B (5 parts by weight) were dissolved in "Solvesso" 150 (80 parts by weight) to give an emulsifiable concentrate which may be diluted with water to the required concentration to give an aqueous emulsion which may be applied by spraying.

("Solvesso" is a Trade Mark and "Solvesso" 150 is a high boiling point aromatic petroleum fraction).

d) Dispersible Powder

Compound No. 23 (10 parts by weight), "Matexil" DA/AC (3 parts by weight), "Aerosol" OT/B (1 part by weight) and china clay 298 (8 parts by weight) were blended and then milled to give a powder composition having a particle size below 50 microns.

"Matexil" is a Trade Mark and "Matexil" DA/AC is the disodium salt of a naphthalenesulfonic acid-formaldehyde condensate; "Aerosol" is a Trade Mark and "Aerosol" OT/B is a formulation of the dioxtyl este of sodium sulfosuccinic acid).

e) Dusting Powder

Compound No. 175 (10 parts by weight), attapulgite (10 parts by weight) and pyrophyllite (80 parts by weight) were thoroughly blended and then ground in a hammer-mill to produce a powder of particle size less than 200 microns.

Emulsifiable concentrates and/or suspensions of the compounds of the invention were prepared essentially as described in part a), b) or c) above and then diluted with water, optionally containing surface active agent and/or oil, to give aqueous compositions of the required concentration which were used, as described in Examples 12 and 13, in the evaluation of the pre-emergence and post-emergence herbicidal activity of the compounds.

Example 12

The pre-emergent herbicidal activity of the compounds of the invention formulated as described in Example 11 was assessed by the following procedure:

The seeds of the test species were sown in rows 2 cm deep in soil contained in seed boxes. The monocotyledonous plants and the dicotyledonous plants were sown in separate boxes and after sowing the two boxes were sprayed with the required quantity of a composition of the invention. Two duplicate seed boxed were prepared in the same manner but were not sprayed with a composition of the invention and were used for comparison purposes. All the boxes were placed in a glass house, lightly watered with an overhead spray to initiate germination and then sub-irrigated as required for optimum plant growth. After three weeks the boxes were removed from the glass house and the effect of the treatment was visually assessed. The results are presented in Table 3 where the damage to plants is rated on a scale of from 0 to 5 where 0 represents from 0 to 10% damage, 1 represents from 11 to 30% damage, 2 represents from 31 to 60% damage, 3 represents from 61 to 80% damage, 4 represents from 81 to 99% damage and 5 represents 100% kill. A dash (-) means that no experiment was carried out.

The names of the test plants are as follows:

Wh Wheat
Ot Wild Oats
Rg Ryegrass
Jm Japanese millet
B Barley
P Peas
Ip Ipomea
Ms Mustard
Sf Sunflower

Pre-emergent Herbicidal Activity

| Compound No. | Application Rate Kg/ha | TEST PLANT | | | | | | | | |
|---|---|---|---|---|---|---|---|---|---|---|
| | | Wh | Jm | Rg | Ot | B | P | Ip | Ms | Sf |
| 2 | 0.40 | 0 | 2 | 0 | 0 | 0 | 0 | 3 | 0 | 1 |
| 6 | 0.40 | 3 | 0 | 1 | 0 | 1 | 1 | 2 | 1 | 2 |
| 6 | 0.10 | - | - | - | - | - | 2 | 0 | 0 | 1 |
| 6 | 0.025 | - | - | - | - | - | 1 | 0 | 0 | 1 |
| 7 | 0.40 | 1 | 3 | 0 | 0 | 2 | 3 | 3 | 4 | 4 |
| 7 | 0.10 | - | - | - | - | - | 0 | 0 | 3 | 4 |
| 7 | 0.025 | - | - | - | - | - | 0 | 0 | 2 | 0 |
| 19 | 0.40 | 1 | 2 | 0 | 2 | 3 | 3 | 4 | 5 | 4 |
| 19 | 0.10 | - | - | - | - | - | 0 | 3 | 4 | 4 |
| 19 | 0.025 | - | - | - | - | - | 1 | 2 | 3 | 2 |
| 20 | 0.40 | 2 | 1 | 0 | 0 | 2 | 4 | 3 | 4 | 4 |
| 20 | 0.10 | - | - | - | - | - | 2 | 2 | 3 | 4 |
| 23 | 0.40 | 4 | 4 | 1 | 0 | 4 | 4 | 2 | 4 | 4 |
| 23 | 0.10 | - | - | - | - | - | 2 | 0 | 4 | 4 |
| 24 | 0.40 | 0 | 4 | 0 | 0 | 3 | 4 | 5 | 5 | 5 |
| 24 | 0.10 | - | - | - | - | - | 1 | 5 | 4 | 5 |
| 24 | 0.025 | - | - | - | - | - | 0 | 0 | 3 | 3 |
| 27 | 0.40 | 1 | 2 | 0 | 0 | 0 | 0 | 2 | 4 | 4 |
| 27 | 0.10 | - | - | - | - | - | 0 | - | 4 | 4 |
| 28 | 0.40 | 0 | 0 | 0 | 0 | 0 | 1 | 1 | 4 | 3 |
| 31 | 0.40 | 0 | 0 | 0 | 0 | 1 | 0 | 1 | 4 | 4 |
| 32 | 0.40 | 0 | 0 | 0 | 0 | 0 | - | 3 | 3 | 4 |
| 32 | 0.10 | - | - | - | - | - | - | 3 | 1 | 4 |

TABLE 3 (Continued)

Pre-emergent Herbicidal Activity

| Compound No. | Application Rate Kg/ha | TEST PLANT | | | | | | | | |
|---|---|---|---|---|---|---|---|---|---|---|
| | | Wh | Jm | Rg | Ot | B | P | Ip | Ms | Sf |
| 132 | 0.40 | 0 | 0 | 0 | 0 | 0 | 1 | 5 | 2 | 5 |
| 171 | 0.40 | 0 | 0 | 0 | 0 | 0 | 0 | 2 | 4 | 4 |
| 171 | 0.10 | - | - | - | - | - | 0 | 1 | 3 | 2 |
| 172 | 0.40 | 0 | 0 | 0 | 0 | 0 | 3 | 3 | 4 | 4 |
| 172 | 0.10 | - | - | - | - | - | 0 | 0 | 2 | 3 |
| 175 | 0.40 | 3 | 0 | 0 | 0 | 0 | 0 | 5 | 4 | 5 |
| 175 | 0.10 | - | - | - | - | - | 0 | 1 | 3 | 4 |
| 175 | 0.025 | - | - | - | - | - | 0 | 2 | 3 | 0 |
| 176 | 0.40 | 0 | 1 | 0 | 0 | 1 | - | 2 | 2 | 3 |

Example 13

The post-emergent herbicidal activity of the compounds of the invention formulated as described in Example 11 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glass house, lightly watered with an overhead spray to initiate germination and then sub-irrigated as required for optimum plant growth. After the plants had grown to a height of about 10 to 12.5 cm, one box of each of the monocotyledonous plants and the dicotyledonous plants was removed from the glass house and sprayed with the required quantity of a composition of the invention. After spraying the boxes were returned to the glass house for a further 3 weeks and the effect of treatment was visually assessed by comparison with the untreated controls. The results are presented in Table 4 where the damage to plants is rated on a scale o from 0 to 5 where 0 represents from 0 to 10% damage, 1 represents from 11 to 30% damage, 2 represents from 31 to 60% damage, 3 represents from 61 to 80% damage, 4 represents from 81 to 99% damage and 5 represents 100% kill. A dash (-) means that no experiment was carried out.

The names of the test plants are as follows:

Wh Wheat
Ot Wild Oats
Rg Ryegrass
Jm Japanese millet

B Barley
P Peas
Ip Ipomea
Ms Mustard
Sf Sunflower
Mz Maize

TABLE 4

Post-emergent Herbicidal Activity

| Compound No. | Application Rate kg/ha | TEST PLANT | | | | | | | | | |
|---|---|---|---|---|---|---|---|---|---|---|---|
| | | Wh | Jm | Rg | Ot | B | P | Ip | Ms | Sf | Mz |
| 1 | 0.40 | 0 | 0 | 0 | 0 | 1 | 2 | 0 | 2 | 5 | 0 |
| 1 | 0.10 | - | - | - | - | - | 1 | 0 | 2 | 3 | - |
| 2 | 0.40 | 0 | 0 | 0 | 0 | 0 | 2 | 0 | 2 | 4 | 0 |
| 5 | 0.40 | 0 | 0 | 0 | 0 | 1 | 1 | 4 | 5 | 5 | 0 |
| 5 | 0.10 | 0 | 0 | 0 | 0 | 0 | 0 | 3 | 5 | 3 | 0 |
| 5 | 0.025 | 0 | 0 | 0 | 0 | 0 | 0 | 3 | 3 | 3 | 0 |
| 6 | 0.40 | 3 | 2 | 1 | 3 | 3 | 3 | 5 | 5 | 5 | 4 |
| 6 | 0.10 | - | - | - | - | - | 2 | 3 | 5 | 5 | - |
| 6 | 0.025 | - | - | - | - | - | 1 | 3 | 3 | 3 | - |
| 7 | 0.40 | 1 | 3 | 0 | 0 | 2 | 5 | 4 | 4 | 5 | 0 |
| 7 | 0.10 | - | - | - | - | - | 3 | 3 | 4 | 4 | - |
| 7 | 0.025 | - | - | - | - | - | 3 | 1 | 2 | 3 | - |
| 19 | 0.40 | 2 | 3 | 2 | 2 | 4 | 2 | 4 | 4 | 5 | 1 |
| 19 | 0.10 | - | - | - | - | - | 2 | 3 | 4 | 4 | - |
| 19 | 0.025 | - | - | - | - | - | 1 | 3 | 3 | 3 | - |
| 20 | 0.40 | 0 | 0 | 0 | 0 | 0 | 3 | 3 | 4 | 4 | 0 |
| 20 | 0.10 | 0 | 0 | 0 | 0 | 0 | 2 | 1 | 4 | 2 | 0 |
| 23 | 0.40 | 0 | 1 | 0 | 0 | 2 | 3 | 3 | 5 | 5 | 2 |
| 23 | 0.10 | 0 | 0 | 0 | 0 | 0 | 2 | 2 | 3 | 4 | 0 |
| 23 | 0.025 | 0 | 0 | 0 | 0 | 0 | 1 | 1 | 3 | 4 | 0 |
| 24 | 0.40 | 0 | 0 | 0 | 0 | 0 | 3 | 4 | 5 | 5 | 0 |
| 24 | 0.10 | 0 | 0 | 0 | 0 | 0 | 1 | 2 | 5 | 5 | 0 |
| 24 | 0.025 | 0 | 0 | 0 | 0 | 0 | 0 | 1 | 3 | 4 | 0 |
| 27 | 0.40 | 0 | 3 | 0 | 0 | 2 | 3 | 3 | 4 | 4 | 0 |
| 27 | 0.10 | - | - | - | - | - | 1 | 2 | 4 | 3 | - |
| 28 | 0.40 | 0 | 3 | 0 | 0 | 2 | 3 | 3 | 4 | 4 | 0 |
| 28 | 0.10 | - | - | - | - | - | 1 | 1 | 3 | 2 | - |

TABLE 4 (Continued)

Post-emergent Herbicidal Activity

| Compound No. | Application Rate kg/ha | TEST PLANT | | | | | | | | | |
|---|---|---|---|---|---|---|---|---|---|---|---|
| | | Wh | Jm | Rg | Ot | B | P | Ip | Ms | Sf | Mz |
| 31 | 0.40 | 0 | 0 | 0 | 0 | 0 | 1 | 1 | 4 | 3 | 2 |
| 31 | 0.10 | - | - | - | - | - | 1 | 1 | 5 | 2 | - |
| 32 | 0.40 | 0 | 0 | 0 | 0 | 0 | 2 | 1 | 5 | 4 | 0 |
| 32 | 0.10 | - | - | - | - | - | 2 | 1 | 3 | 4 | 0 |
| 34 | | | | | | | | | | | |
| 171 | 0.40 | 1 | 1 | 0 | 0 | 0 | 2 | 3 | 3 | 5 | 0 |
| 171 | 0.10 | - | - | - | - | - | 3 | 3 | 3 | 4 | - |
| 172 | 0.40 | 1 | 2 | 0 | 0 | 1 | 3 | 3 | 5 | 5 | 0 |
| 172 | 0.10 | - | - | - | - | - | 3 | 1 | 4 | 4 | - |
| 172 | 0.025 | - | - | - | - | - | 2 | 0 | 1 | 3 | - |
| 175 | 0.40 | 2 | 1 | 0 | 0 | 2 | 2 | 3 | 3 | 3 | 4 |
| 175 | 0.10 | - | - | - | - | - | 1 | 3 | 3 | 2 | - |
| 176 | 0.40 | 0 | 0 | 0 | 0 | 0 | 3 | 3 | 4 | 4 | 0 |
| 176 | 0.10 | - | - | - | - | - | 2 | 2 | 3 | 4 | - |

## Example 14

The compounds were formulated for test by mixing an appropriate amount with 5 ml of an emulsion prepared by diluting 160 ml of a solution containing 21.9 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsin containing a test compound was then diluted to 40 ml with water and sprayed on to young pot plants (post-emergence test) of the species named in Tables 5 and 6 below. Damage to test plants was assessed after 13 days. In Table 5, assessment was on a scale of 0 to 5 wherein 0 is 0 to 20% damage and 5 is complete kill. In Table 6, assessment was on a 0 to 9 scale where 0 represents zero damage,
1 represents from 1 to 5% damage,
2 represents from 6 to 15% damage,
3 represents from 16 to 25% damage
4 represents from 26 to 35% damage,
5 represents from 36 to 59% damage,

6 represents from 60 to 69% damage,
7 represents from 70 to 79% damage,
8 represents from 80 to 89% damage and
9 represents from 90 to 100% damage.

The degree of herbicidal damage was assessed by comparison with untreated control plants. The results are given in Table 5 and 6 below. A dash (-) means no experiment was carried out.

The names of the test plants were as follows:

Sb Sugar Beet
Rp Oilseed Rape
Ct Cotton
Sy Soya Bean
Mz Maize
Ww Winter Wheat
Rc Rice
Bd Bidens pilosa
Ip Ipomea
Am Amaranthus retroflexus
Pi Polygonum
Ca Chenopodium album
Ga Galium aparine
Xs Xanthium spinosum
Ab Abutilon theophrasti
Co Cassia obtusifolia
Av Avena fatua
Dg Digitaria sanguinalis
Al Alopecurus myosuroides
StSetaria viridis
Ec Echinochloa crus-galli
Sh Sorghum halepense
Ag Agropyron repens
Cn Cyperus rotundus
Ce Cyperus esculentus
Eh Euphorbia heterophylla

# Table 5

## Post-Emergent Herbicidal Activity

| Compound No. | 6 | 6 | 6 | 6 | 7 | 7 | 7 | 7 | 19 | 19 | 19 | 31 | 31 | 171 | 171 | 171 | 172 | 172 | 172 | 175 |
|---|---|---|---|---|---|---|---|---|---|---|---|---|---|---|---|---|---|---|---|---|
| Rate g/ha | 625 | 125 | 25 | 5 | 625 | 125 | 25 | 5 | 625 | 125 | 25 | 625 | 125 | 625 | 125 | 25 | 625 | 125 | 25 | 625 |
| Test Plants | | | | | | | | | | | | | | | | | | | | |
| Sb | 4 | 4 | 4 | 3 | 5 | 3 | 3 | 2 | 3 | 3 | 1 | 3 | 2 | 4 | 3 | 3 | 4 | 3 | 2 | 2 |
| Rp | 4 | 4 | 4 | 2 | 5 | 5 | 4 | 3 | 4 | 4 | 2 | 4 | 4 | 4 | 4 | 4 | 4 | 4 | 4 | 3 |
| Ct | 4 | 3 | 4 | 2 | 4 | 3 | 2 | 1 | 2 | 1 | 0 | 3 | 3 | 4 | 3 | 2 | 2 | 3 | 1 | 1 |
| Sy | 5 | 5 | 1 | 0 | 4 | 4 | 4 | 0 | 3 | 2 | 0 | 1 | 0 | 4 | 4 | 1 | 4 | 3 | 2 | 0 |
| Mz | 4 | 2 | 1 | 1 | 2 | 0 | 0 | 0 | 4 | 4 | 2 | 2 | 2 | 1 | 1 | 0 | 1 | 1 | 0 | 3 |
| Ww | 4 | 2 | 0 | 0 | 2 | 1 | 2 | 1 | 3 | 3 | 3 | 3 | 1 | 2 | 1 | 0 | 1 | 1 | 1 | 0 |
| Rc | 1 | 0 | 0 | 0 | 2 | 1 | 1 | 0 | 1 | 1 | 1 | 1 | 1 | 0 | 1 | 1 | 1 | 1 | 1 | 0 |
| Bd | 5 | 5 | 5 | 4 | 4 | 4 | 3 | 2 | 4 | 4 | 2 | 5 | 4 | 4 | 4 | 3 | 4 | 4 | 3 | 4 |
| Ip | 4 | 4 | 4 | 4 | 4 | 3 | 3 | 2 | 4 | 4 | 2 | 3 | 2 | 3 | 4 | 3 | 4 | 3 | 1 | 4 |
| Am | 5 | 4 | 3 | 2 | 4 | 4 | 3 | 3 | 4 | 4 | 3 | 1 | 1 | 3 | 3 | 2 | 4 | 3 | 3 | 4 |
| Pi | 4 | 4 | 4 | 4 | 4 | 4 | 4 | 3 | 3 | 3 | 2 | 2 | 0 | 3 | 2 | 1 | 4 | 3 | 2 | 1 |
| Ca | 5 | 4 | 3 | 1 | 3 | 3 | 1 | 2 | 4 | 4 | 2 | 2 | 1 | 3 | 1 | 1 | 3 | 1 | 1 | 4 |
| Ga | 5 | 5 | 4 | 3 | 4 | 4 | 3 | 2 | 3 | 2 | 1 | 4 | 2 | 4 | 4 | 3 | 4 | 3 | 3 | 3 |

Table 5 (Continued)

Post-Emergent Herbicidal Activity

| Compound No. | 6 | 6 | 6 | 6 | 7 | 7 | 7 | 7 | 19 | 19 | 19 | 31 | 31 | 171 | 171 | 171 | 172 | 172 | 172 | 175 |
|---|---|---|---|---|---|---|---|---|---|---|---|---|---|---|---|---|---|---|---|---|
| Rate g/ha | 625 | 125 | 25 | 5 | 625 | 125 | 25 | 5 | 625 | 125 | 25 | 625 | 125 | 625 | 125 | 25 | 625 | 125 | 25 | 625 |
| Test Plants | | | | | | | | | | | | | | | | | | | | |
| Xs | 5 | 5 | 5 | 3 | 5 | 4 | 3 | 2 | 4 | 4 | 1 | 5 | 5 | 4 | 4 | 3 | 5 | 4 | 3 | 2 |
| Ab | 5 | 5 | 3 | 3 | 5 | 4 | 3 | 0 | 3 | 1 | 0 | 4 | 4 | 3 | 3 | 2 | 5 | 3 | 2 | 1 |
| Co | 1 | 0 | 0 | 0 | 2 | 1 | 0 | 1 | 1 | 1 | 0 | 0 | 0 | 1 | 1 | 0 | 1 | 1 | 1 | 0 |
| Av | 4 | 4 | 1 | 0 | 3 | 3 | 1 | 3 | 4 | 3 | 2 | 4 | 2 | 3 | 2 | 3 | 2 | 3 | 2 | 2 |
| Dg | 4 | 2 | 1 | 2 | 2 | 1 | 0 | 2 | 4 | 3 | 0 | 1 | 0 | 2 | 1 | 0 | 0 | 1 | 0 | 2 |
| Al | 3 | 3 | 1 | 0 | 3 | 3 | 2 | 3 | 3 | 2 | 2 | 3 | 3 | 3 | 2 | 2 | 3 | 2 | 2 | 1 |
| St | 5 | 4 | 1 | 0 | 3 | 3 | 2 | 0 | 3 | 2 | 0 | 2 | 1 | 3 | 3 | 1 | 2 | 2 | 1 | 0 |
| Ec | 5 | 4 | 3 | 2 | 4 | 4 | 2 | 1 | 4 | 2 | 1 | 3 | 0 | 3 | 2 | 2 | 2 | 2 | 1 | 2 |
| Sh | 2 | 2 | 1 | 0 | 3 | 2 | 1 | 1 | 3 | 2 | 1 | 4 | 0 | 3 | 3 | 2 | 2 | 1 | 2 | 4 |
| Ag | 2 | 3 | 2 | 0 | 4 | 3 | 2 | 2 | 2 | 2 | 2 | 3 | 3 | 3 | 0 | 0 | 2 | 1 | 0 | 1 |
| Cn | 4 | 4 | 0 | 0 | 4 | 4 | 3 | 0 | 1 | 1 | 1 | 0 | 0 | 2 | 3 | 1 | 3 | 3 | 2 | 1 |

## Table 6

## Post-Emergent Herbicidal Activity

| Compound No. | 5 | 5 | 5 | 20 | 23 | 23 | 23 | 24 | 24 | 27 | 27 | 28 | 28 |
|---|---|---|---|---|---|---|---|---|---|---|---|---|---|
| Rate g/ha | 400 | 100 | 25 | 400 | 400 | 100 | 25 | 400 | 100 | 400 | 100 | 400 | 100 |
| Test Plants | | | | | | | | | | | | | |
| Sb | 8 | 4 | 3 | 5 | 6 | 0 | 0 | | | 0 | 0 | 5 | 0 |
| Rp | 9 | 9 | 6 | 8 | 9 | 9 | 7 | | | 7 | 6 | 6 | 9 |
| Ct | 5 | 6 | 0 | - | 5 | 5 | 2 | | | 0 | 0 | 0 | 0 |
| Sy | 2 | 3 | 2 | 0 | 2 | 0 | 0 | | | 2 | 0 | 0 | 0 |
| Mz | 5 | 2 | 0 | 1 | 5 | 0 | 0 | | | 2 | 0 | 0 | 0 |
| Ww | 7 | 3 | 0 | 0 | 7 | 5 | 1 | | | 7 | 5 | 0 | 0 |
| Rc | 4 | 0 | 0 | 0 | 2 | 0 | 3 | | | 0 | 0 | 0 | 3 |
| Bd | 8 | 6 | 5 | 6 | 9 | 4 | 3 | | | 7 | 2 | 6 | 8 |
| Ip | 7 | 6 | 0 | 5 | 6 | 6 | 5 | | | 5 | 1 | 5 | 4 |
| Am | 6 | 5 | 4 | 8 | 5 | 0 | 0 | | | 6 | 6 | 5 | 7 |
| Pi | 7 | 5 | 0 | 7 | 5 | 0 | 0 | | | 7 | 2 | 8 | 6 |
| Ca | 7 | 5 | 3 | 5 | 3 | 0 | 0 | | | 6 | 7 | 0 | 3 |
| Ga | 8 | 6 | 3 | 7 | 9 | 7 | 4 | | | 7 | 2 | 7 | 7 |

Table 6 (continued)

Post Emergent Herbicidal Activity

| Compound No. | 5 | 5 | 5 | 20 | 23 | 23 | 23 | 24 | 24 | 27 | 27 | 28 | 28 |
|---|---|---|---|---|---|---|---|---|---|---|---|---|---|
| Rate g/ha | 400 | 100 | 25 | 400 | 400 | 100 | 25 | 400 | 100 | 400 | 100 | 400 | 100 |
| Test Plants | | | | | | | | | | | | | |
| Xs | 8 | 7 | 6 | 8 | 0 | 5 | 4 | | | 5 | 3 | 5 | 5 |
| Ab | 7 | 7 | 6 | 5 | 7 | 6 | 5 | | | - | 5 | 6 | 2 |
| Eh | 6 | 5 | 0 | 0 | 3 | 0 | 0 | | | 0 | 0 | 1 | 0 |
| Av | 6 | 2 | 2 | 5 | 6 | 5 | 0 | | | 5 | 0 | 2 | 0 |
| Dg | 0 | 0 | 0 | 0 | 0 | 0 | 0 | | | 0 | 0 | 7 | 0 |
| Al | 6 | 2 | 2 | 4 | 9 | 0 | 0 | | | 5 | 4 | 6 | 5 |
| St | 7 | 0 | 0 | 0 | 6 | 5 | 0 | | | 2 | 0 | 0 | 0 |
| Ec | 7 | 0 | 0 | 7 | 7 | 7 | 6 | | | 8 | 0 | 8 | 0 |
| Sh | 6 | 2 | 0 | 0 | 6 | 0 | 0 | | | 7 | 0 | 7 | 4 |
| Ag | 7 | 5 | 5 | 0 | 6 | 7 | 7 | | | 7 | 7 | 0 | 7 |
| Ce | 6 | 0 | 0 | 5 | 0 | 0 | 0 | | | 0 | 0 | 0 | 5 |

## Claims

1. A compound of formula I and the salts thereof

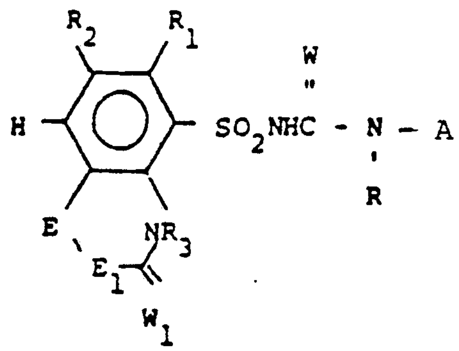

I

wherein

W and $W_1$ are independently O or S,

E is O, S(O)m, or N-$R_4$

$E_1$ is $CH_2$, $CH_2CH_2$, CH($C_1$-$C_4$ alkyl), $C(CH_3)_2$ or CH(aryl);

$R_1$ and $R_2$ are independently selected from hydrogen, halogen, $C_1$-$C_4$ alkyl, $C_1$-$C_4$ haloalkyl, arylalkyl, nitro, $C_1$-$C_4$ alkoxy, $C_1$-$C_4$ haloalkoxy, cyano, carboxy, carbamoyl, N-($C_1$-$C_4$ alkyl)carbamoyl, N,N-di($C_1$-$C_4$ alkyl)-carbamoyl $C_1$-$C_4$ alkoxycarbonyl, $C_1$-$C_4$ alkylthio, $C_1$-$C_4$ alkylsulfinyl, $C_1$-$C_4$ alkylsulfonyl, sulfamoyl, $C_1$-$C_4$ alkylsulfamoyl, di($C_1$-$C_4$ alkyl) sulfamoyl, amino, $C_1$-$C_4$ alkylamino or di($C_1$-$C_4$ alkyl) amino; $C_1$-$C_4$ acylamino;

R, $R_3$ and $R_4$ are each independently selected from hydrogen, optionally substituted $C_1$-$C_4$ alkyl, $C_2$-$C_4$ alkenyl, $C_2$-$C_4$ alkynyl, or optionally substituted aryl or arylalkyl and m = 0, 1 or 2;

A is 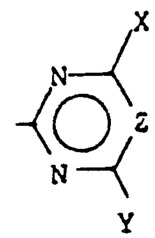 , 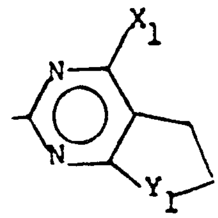 , 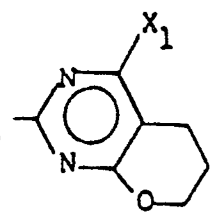 , 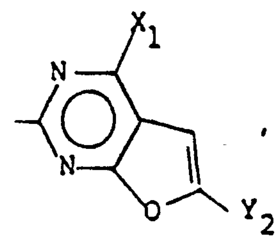 ,

A-1 A-2 A-3 A-4

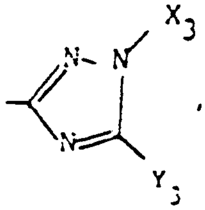 , 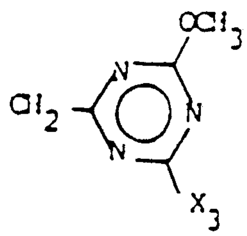 or 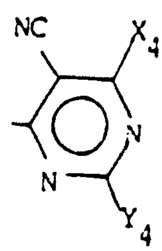

A-5 A-6 A-7

X is $CH_3$, $OCH_3$, $OCH_2CH_3$, Cl, F, Br, I, $OCF_2H$, $CH_2F$ or $CF_3$; Y is H , $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $N(OCH_3)CH_3$, $OCH_2CH$-$CH_2$, $OCH_2C$-$CH$, $OCH_2CF_3$, cyclopropyl, $OCH_2CH_2OCH_3$, $CH_2SCH_3$, (C=O)$R_4$, $CR(QCH_3)_2$,

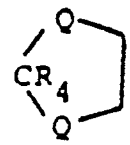

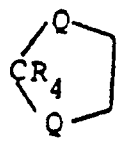 , 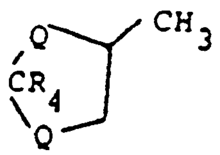

$CR_4(QCH_2CH_3)_2$, $OCF_2H$, $SCF_2H$, C = CH or C = $CCH_3$ where Q is O or S and $R_4$ is H or $CH_3$;
Z is CH, N, $CCH_3$, $CC_2H_5$, CCl or CBr;
$Y_1$ is O or $CH_2$;
$X_1$ is $CH_3$, $OCH_3$, $OC_2H_5$ OR $OCF_2H$;
$Y_2$ is H or $CH_3$;
$X_2$ is $CH_3$, $C_2H_5$ OR $CH_2CF_3$;
$Y_3$ is $OCH_3$, $OC_2H_5$, $SCH_3$, $SC_2H_5$, $CH_3$ or $CH_2CH_3$;
$X_3$ is $CH_3$ or $OCH_3$;
$Y_4$ is $CH_3$, $OCH_3$, $OCH_2CH_3$ or Cl; and
$X_4$ is $CH_3$, $OCH_3$, $OCH_2CH_3$, $CH_2OCH_3$ or Cl.

2. A compound according to claim 1, wherein $E_1$ is selected from $CH_2$, $CH_2CH_2$, CH($C_1$-$C_4$ alkyl) or C-$(CH_3)_2$.

3. A compound according to claim 1 or claim 2, wherein W is O or S and A is A-1.

4. A compound according to any one of claims 1-3, wherein
R and $R_2$ are hydrogen;
$R_1$ is hydrogen, fluorine, chlorine, bromine, methyl, methoxy, trifluoromethyl methoxycarbonyl or methylthio; and Y is methyl, methoxy, ethoxy, methoxymethyl, trifluoromethyl, 2,2,2-trifluoroethoxy, dimethoxymethyl, or difluoromethoxy.

5. A compound according to claim 1 or claim 2 wherein:
E is O, S, NH or $NCH_3$;
$E_1$ is $CH_2$, $CH_2CH_2$, or $CH(CH_3$;
$W_1$ is O or S, and $R_3$ is methyl or ethyl.

6. A compound of formula II

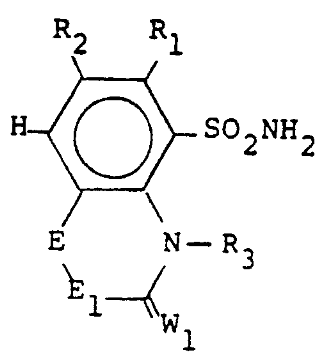

II

wherein $R_1$, $R_2$, $R_3$, $W_1$, E and $E_1$ are as defined in claim 1.

7. A process for the preparation of a compound according to claim 1 by the reaction of a sulfonylisocyanate or a sulfonylisothiocyanate of formula X with a heterocyclic amine of formula IX.

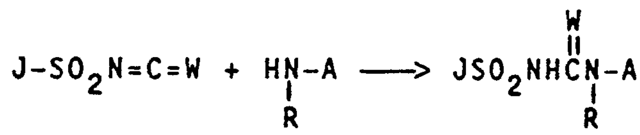

X IX I

where J represents the bicyclic system

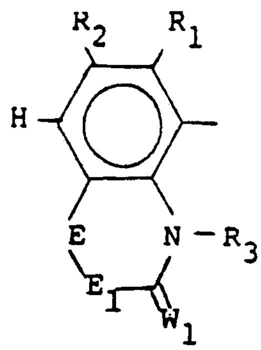

and W, A and R are as defined in claim 1.

8. A process for the preparation of a compound according to claim 1, where W is oxygen, by the reaction of a phenyl carbamate of formula XI with an amine of formula IX.

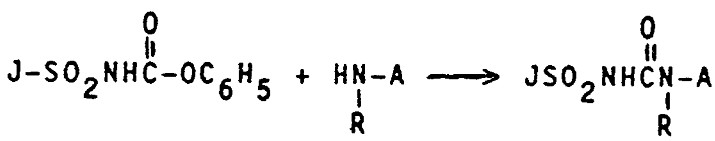

XI IX I

wherein A and J are as defined in claim 7.

9. A process for the preparation of a compound according to claim 1, where W is oxygen, by the reaction of a heterocyclic carbamate of formula XII with a sulfonamide of formula II.

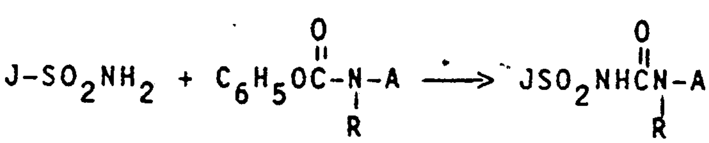

II XII I

wherein A and J are as defined in claim 7.

10. A herbicidal composition comprising a compound according to claim 1.

11. A process for severely damaging or killing unwanted plants which process comprises applying to the plants, or to the growth medium of the plants, an effective amount of a compound according to claim 1.

12. A process for controlling weeds in cultivated crops which process comprises applying to the crop, or to the growth medium of the crop, a compound according to claim 1 in an amount sufficient to severely damage or kill the weeds but insufficient to damage the crop substantially.

13. A process for regulating the growth of a plant which process comprises applying to the plant, to the

seed of the plant, or to the growth medium of the plant, an effective amount of a compound according to claim 1.

14. A plant growth regulating composition comprising a compound according to claim 1 and an inert carrier therefor.

15. A herbicidal compositon comprising a mixture of at least one compound according to claim 1 with at least one other herbicide.

16. A herbicidal composition according to claim 15 wherein the other herbicide has a complementary action to that of the compound according to claim 1.

17. A herbicidal composition according to claim 15 wherein the other herbicide is a contact herbicide.

European Patent Office

# EUROPEAN SEARCH REPORT

Application Number

EP 90 30 2188

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | EP-A-0 082 681 (E.I. DU PONT DE NEMOURS AND CO.)<br>* Pages 167-185; claims 1,31 *<br>--- | 1 | C 07 D 413/12<br>A 01 N 47/36<br>C 07 D 417/12<br>C 07 D 403/12<br>C 07 D 265/36 |
| A | EP-A-0 168 135 (E.I. DU PONT DE NEMOURS AND CO.)<br>* Claim 1 *<br>----- | 1 | |
| | | | **TECHNICAL FIELDS SEARCHED (Int. Cl.5)** |
| | | | C 07 D 413/00<br>C 07 D 403/00<br>C 07 D 417/00 |

The present search report has been drawn up for all claims

| Place of search | Date of completion of the search | Examiner |
|---|---|---|
| THE HAGUE | 25-04-1990 | DE JONG B.S. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)